# EUROPÄISCHE PATENTSCHRIFT

(11) **EP 0 372 305 B1**
(45) Veröffentlichungstag und Bekanntmachung des Hinweises auf die Patenterteilung: **04.08.1993**
(21) Anmeldenummer: 89121681.4
(22) Anmeldetag: 23.11.1989
(51) Int. Cl.: C07D 403/12, C07D 237/22, C07D 417/14, A61K 31/50

(54) **Neue Piperazinylalkyl-3(2H)-pyridazinone, Verfahren zu ihrer Herstellung und ihre Verwendung als blutdrucksenkende Mittel**
Piperazinylalkyl-3(2H)-pyridazinones, method for their preparation and their use as antihypertensive agents
Pipérazinylalkyl-3(2H)-pyridazinones, leur procédé de préparation et leur application comme agents antihypertensifs

(30) Priorität: 06.12.1988 AT 2991/88
(43) Veröffentlichungstag der Anmeldung: 13.06.1990
(73) Patentinhaber: Hafslund Nycomed Pharma Aktiengesellschaft, A-4021 Linz (AT)
(72) Erfinder: Blaschke, Heinz, Dr., A-4020 Linz (AT); Stroissnig, Heimo, Dr., A-1180 Wien (AT); Fellier, Harald, Dr., A-4040 Puchenau (AT); Enzenhofer, Rita, Dr., A-4100 Ottensheim (AT)
(74) Vertreter: Kunz, Ekkehard, Dr.

(56) Entgegenhaltungen:
- EP-A- 0 201 765
- EP-A- 0 275 997
- DE-A- 1 942 405
- DE-A- 2 334 009
- FR-A- 2 124 164
- CHEMICAL ABSTRACTS, Band 106, Nr. 1, 5. Jänner 1987, Columbus, Ohio, US; GOMEZ GIL, M. et al. "3(2H)- pyridazinones" Seite 477, Spalte 1, Zusammen-fassung-Nr. 5 064r
- CHEMICAL ABSTRACTS, Band 109, Nr. 15, 10. Oktober 1988, Columbus, Ohio, US; TAKAYA, MASHIRO "Studies on pyridazinone derivatives. XII.Synthesis and analgesic activity of 2-phenyl-3(2H)- pyridazinone derivatives" Seite 685, Spalte 1, Zusammen-fassung-Nr. 128 932s
- CHEMICAL ABSTRACTS, Band 105, Nr. 5, 4. August 1986, Columbus, Ohio, US; OKUJIMA, HIROMI et al.: "Pyridazinones." Seite 742, Spalte 2, Zusammen-fassung-Nr. 42 832q

## Beschreibung

Die vorliegende Erfindung betrifft neue Piperazinylalkyl-3(2H)-pyridazinone und ihre pharmazeutisch verwendbaren Salze, Verfahren zu ihrer Herstellung und ihre Verwendung als blutdrucksenkende Mittel.

Es ist bekannt, daß Alpha-Rezeptoren-Blocker als blutdrucksenkende Mittel verwendet werden können. So werden beispielsweise in der DE-PS 19 42 405 arylsubstituierte Piperazinylpropylenamino-uracile beschrieben, die blutdrucksenkend wirken und den durch Adrenalin und Noradrenalin an der despinalisierten Ratte hervorgerufenen blutdrucksteigernden Effekt blockieren.

Gegenstand der Erfindung sind neue Piperazinylalkyl-3(2H)-pyridazinone der Formel 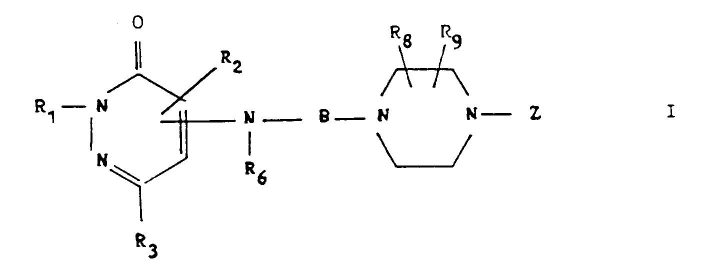
in der die Reste
R₁ Wasserstoff, Phenyl, Benzyl, unsubstituiertes oder ein- oder mehrfach durch Hydroxy, Piperidin, Morpholin oder durch eine Gruppe NR₄R₅, in der R₄ und R₅ gleich oder verschieden sein können und Wasserstoff, Methyl oder Ethyl darstellen, substituiertes (C₁ - C₆)-Alkyl,
R₂ und R₃ Wasserstoff, Halogen, (C₁ - C₆)-Alkoxy oder (C₁ - C₆)-Alkyl, wobei mindestens einer der Reste R₂ oder R₃ Wasserstoff bedeutet,
R₆ Wasserstoff, (C₁ - C₄)-Alkyl, Phenyl, Benzyl oder Phenylethyl,
B unsubstituiertes oder ein- oder mehrfach durch Hydroxy, (C₁ - C₄)-Alkyl oder durch die Gruppe NR₄R₅ substituiertes (C₁ - C₇)-Alkylen, welches gegebenenfalls zu einem alicyclischen 4 bis 7-Ring geschlossen sein kann,
R₈ und R₉, die gleich oder verschieden sein können, Wasserstoff oder (C₁ - C₆)-Alkyl und
Z unsubstituiertes oder ein- oder mehrfach oder (C₁ - C₆)-Alkyl, (C₁ - C₆)-Alkoxy, Benzyloxy, Trifluormethyl, Halogen, Nitro, (C₃ - C₇)-Cycloalkoxy, (C₁ - C₄)-Alkylthio, Trifluormethylthio oder durch die Gruppe NR₄R₅ substituiertes Phenyl, Naphthyl, Pyridyl oder Thiazolyl darstellen, ihre pharmazeutisch verwendbaren Salze, ein Verfahren zu ihrer Herstellung sowie ihre Verwendung als Medikament zur Behandlung von Hypertonie, Herzinsuffizienz und peripheren Kreislaufstörungen.

Der Ausdruck (C₁ - C₆)-Alkyl umfaßt alle geradkettigen und ein- oder mehrfach verzweigten gesättigten Kohlenwasserstoffreste mit 1 bis 6 Kohlenstoffatomen wie beispielsweise Methyl, Isopropyl, tert. Butyl, Neopentyl, Hexyl und dergleichen.
In der (C₁ - C₆)-Alkoxygruppe besitzt der Alkylrest die obige Bedeutung.
Der Ausdruck (C₁ - C₇)-Alkylen steht für einen divalenten, geradkettigen oder ein- oder mehrfach verzweigten gesättigten Kohlenwasserstoffrest mit 1 bis 7 Kohlenstoffatomen, wobei, falls mindestens 4 Kohlenstoffatome vorhanden sind, die Alkylenkette gegebenenfalls zu einem alicyclischen, gesättigten Ring mit 4 bis 7 Kohlenstoffatomen geschlossen sein kann, wie beispielsweise Cyclobutylen, Cycloheptylen. In der (C₃ - C₇)-Cycloalkoxygruppe besitzt der Cycloalkylrest die Bedeutung eines gesättigten alicyclischen Kohlenwasserstoffrestes mit 3 bis 7 Kohlenstoffatomen.
Unter Halogen ist Fluor, Chlor, Brom oder Jod zu verstehen.

Von den Verbindungen der Formel I sind jene bevorzugt, in denen die Reste
R₁ Wasserstoff, Methyl, Ethyl, tert.-Butyl, Benzyl, 2-Hydroxyethyl oder 2-Dimethylaminoethyl,
R₂ und R₃ Wasserstoff, Chlor, Brom oder Methoxy, wobei mindestens einer der Reste R₂ oder R₃ Wasserstoff bedeutet,
R₆ Wasserstoff, Methyl oder Ethyl,
B geradkettiges (C₂ - C₆)-Alkylen,
R₈ und R₉ Wasserstoff und
Z unsubstituiertes oder ein- oder mehrfach durch Methyl, (C₁ - C₃)-Alkoxy,
Benzyloxy, Trifluormethyl, Fluor, Chlor oder Nitro substituiertes Phenyl oder unsubstituiertes Pyridyl-2 darstellen.

Besonders bevorzugt ist die Verbindung 2-Methyl-4-chlor-5-((2-(4-(2-methoxyphenyl)piperazinyl-1)ethyl)amino)-3(2H)-pyridazinon.

Die Verbindungen der Formel I können, falls R₁ die Bedeutung von Wasserstoff hat, zum Teil oder gänzlich in ihrer tautomeren Form vorliegen. Auch diese tautomeren Formen sind Bestandteil der vorliegenden Erfindung.

Die Verbindungen der Formel I und ihre Salze werden dadurch hergestellt, daß man
a) eine Verbindung der Formel 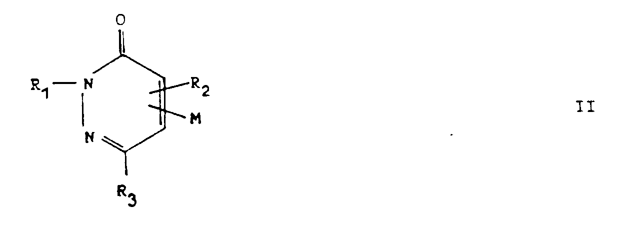 in der R₁, R₂ und R₃ wie oben definiert sind und M eine Abgangsgruppe bedeutet, mit einer Verbindung der Formel 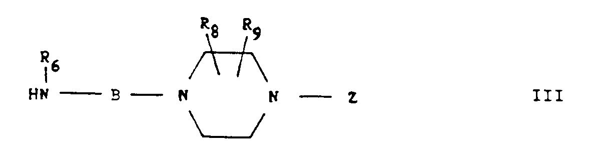 in der R₆,B,R₈,R₉ und Z wie oben definiert sind, umsetzt, oder
b) in einer Verbindung der Formel I, in der einer der Reste R₂ oder R₃ Halogen darstellt und die übrigen Reste wie oben definiert sind, das Halogen von R₂ oder R₃ mittels hydrierender Enthalogenierung durch Wasserstoff ersetzt, oder
c) eine Verbindung der Formel I, in der einer der Reste R₂ oder R₃ Halogen darstellt und die übrigen Reste wie oben definiert sind, mit einem Alkalimetallalkoholat umsetzt, wobei das Halogen von R₂ oder R₃ in einen Rest mit der Bedeutung (C₁ - C₆)-Alkoxy umgewandelt wird, oder
d) in einer Verbindung der Formel I, in der R₁ die Bedeutung von i-Propyl, sec.Butyl, t-Butyl oder Benzyl hat und die übrigen Reste wie oben definiert sind, R₁ durch Säuren abspaltet oder
e) ein Pyridazin der Formel 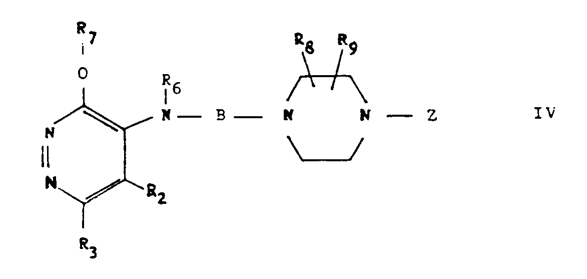 in der R₂, R₃, R₆, B, R₈, R₉ und Z wie oben definiert sind und R₇ die Bedeutung von (C₁ - C₆)-Alkyl hat, unter Etherspaltung mit Säure in das entsprechende 3(2H)-Pyridazinon überführt oder
f) eine Verbindung der Formel I, in der R₁ Wasserstoff und einer der Reste R₂ oder R₃ Halogen darstellt und die übrigen Reste wie oben definiert sind, durch Reaktion mit einem alkylierenden Reagens in Position 2 des Pyridazin-Ringes alkyliert und
g) gewünschtenfalls eine nach a) bis e) erhaltene Verbindung der Formel I in ihre pharmazeutisch verträglichen Salze überführt.

Als Abgangsgruppe M nach Verfahrensvariante a) eignen sich alle üblicherweise verwendeten Abgangsgruppen wie Halogen, p-Toluolsulfonyl, Methansulfonyl oder Trifluormethansulfonyl u.ä. Bevorzugterweise werden Verbindungen der Formel II eingesetzt, in der M die Bedeutung von Chlor oder Brom besitzt.
Die Durchführung der Reaktion nach Verfahrensvariante a) erfolgt in der Weise, daß man eine Verbindung der Formel II oder ihr Tautomeres mit einer Verbindung der Formel III in einem unter Reaktionsbedingungen inerten Verdünnungsmittel bei Temperaturen zwischen etwa 20 und 150 °C oder ohne Lösungsmittel in der Schmelze umsetzt. Als Verdünnungsmittel kommen beispielsweise DMF, DMSO, Acetonitril, Benzol, Toluol, Aceton, Diethylketon, Essigester, Amylacetat, Ethylenglykoldimethylether, Diethylenglykoldimethylether, Tetralin oder Alkohole wie Methanol, Ethanol, Hexanol, Decanol, Dioxan oder Tetrahydrofuran in Frage. Die Reaktionsdauer liegt etwa zwischen 2 und 200 Stunden, wobei höhere Reaktionstemperaturen kürzere Reaktionszeiten bedingen und umgekehrt. Bevorzugte Reaktionsbedingungen sind die Umsetzung der Reaktionspartner in Acetonitril für 5 - 50 Stunden unter Zusatz von mindestens einem Mol Kaliumhydrogencarbonat als säurebindendem Mittel bei Rückflußtemperatur.
Bei der Umsetzung einer Verbindung der Formel II mit einer Verbindung der Formel III entstehen Stellungsisomere, da sich R₂ wie eine Abgangsgruppe verhält. Die Trennung der Stellungsisomeren erfolgt durch übliche Methoden der Chemie, bevorzugt durch Umkristallisation und Säulenchromatographie.

Nach Verfahrensvariante b) wird das Halogen von R₂ oder R₃ mittels hydrierender Enthalogenierung durch Wasserstoff ersetzt. Bevorzugt erfolgt sie in Lösung unter Zusatz eines Katalysators aus Edelmetall oder aus Raney-Nickel. Besonders bewährt hat sich die Verwendung von Palladium auf Kohle als Katalysator. Als Lösungsmittel kommen Alkohole wie Methanol, Ethanol, Hexanol u.ä., Ester wie Methylacetat, Ethylacetat u.ä., Eisessig oder wässrige Salzsäure oder Natronlauge in Frage. Es wird bei für katalytische Hydrierungen üblichen Drücken gearbeit, bevorzugt bei Drücken von Normaldruck bis etwa 5 bar. Die Temperatur kann, abhängig von der verwendeten Verbindung und dem Druck, etwa zwischen 0 °C und 120 °C liegen; bevorzugt wird bei 20 - 70 °C gearbeitet. Die Hydrierung wird bis zur berechneten stöchiometrischen Wasserstoffaufnahme durchgeführt, wobei ein geringer Überschuß an Wasserstoff in den meisten Fällen nicht nachteilig ist.

Nach Verfahrensvariante c) erfolgt die Reaktion mit dem Alkalimetallalkoholat, bevorzugt einem Natriummethylat, in einem polaren Verdünnungsmittel wie DMF, DMSO, in zyklischen Ethern wie Dioxan oder Tetrahydrofuran, in Diethylether, Diethylenglykohldimethylether oder in Alkoholen, wobei die Verwendung des Alkohols, dem das Alkoholat zugrunde liegt, besonders bevorzugt ist. Die Umsetzung erfolgt bei Temperaturen von etwa 80 bis 200 °C und kann erwünschtenfalls in einem Druckgefäß bei Drücken von etwa 2 bis 10 bar durchgeführt werden. Die Reaktonsdauer beträgt in Abhängigkeit von der Art der Ausgangsverbindungen und den Reaktionsparametern, besonders in Abhängigkeit von Druck und Temperatur, etwa 6 bis 120 Stunden. Bevorzugt wird die Reaktion in methanolischer Lösung ohne Druck bei Rückflußtemperatur während 30 - 60 Stunden durchgeführt.

Die Abspaltung von R₁ nach Verfahrensvariante d) kann mittels anorganischer oder organischer Säuren erfolgen. Beispiele für anorganische Säuren sind HCl und HBr, wobei diese Säuren sowohl in wässriger Lösung als auch gelöst in Eisessgig verwendet werden können. Beispiele für organische Säuren sind Trifluoressigsäure, Trifluormethansulfonsäure und Methansulfonsäure. Die Abspaltung erfolgt durch Erwärmen in den genannten Säuren bei Temperaturen von etwa 50 bis 120 °C, bevorzugt bei Rückflußtemperatur; die Reaktionsdauer beträgt abhängig von der Art der Ausgangsverbindungen und den übrigen Reaktionsparametern etwa 0,5 bis 12 Stunden.

Die Umwandlung eines Pyridazins der Formel IV durch Etherspaltung in ein 3(2H)-Pyridazinon der Formel I nach Verfahrensvariante e) kann mit den in Verfahrensvariante d) genannten Säuren erfolgen. Bevorzugt werden Verbindungen der Formel IV eingesetzt, in der R₇ die Bedeutung Methyl besitzt. Die Reaktion wird abhängig von der Art der Ausgangsverbindungen und den übrigen Reaktionsparametern für eine Dauer von 5 Minuten bis 6 Stunden bei etwa 50 - 120 °C, bevorzugt bei der Rückflußtemperatur der eingesetzten Säure durchgeführt.

Die Umsetzung einer Verbindung der Formel I, in der der Rest R₁ Wasserstoff darstellt, nach Verfahrensvariante f) mit einem alkylierenden Reagens erfolgt in wässerig-alkalischer Lösung, wobei als Co-Lösungsmittel Alkohole wie Methanol oder Ethanol, zyklische Ether z.B.: THF, Dioxan, DMF oder DMSO zugesetzt werden können. Die Reaktion kann bei Temperaturen von etwa 20 bis 120 °C, bevorzugt bei 20 bis 70 °C durchgeführt werden. Die Reaktionsdauer beträgt abhängig von der Art der Ausgangsverbindungen und den übrigen Reaktionsparametern 1 bis 12 Stunden, bevorzugt 1 - 4 Stunden.

Die Aufarbeitung der nach Verfahrensvarianten a) bis f) erhaltenen Verbindungen erfolgt in der üblichen Weise durch Eindampfen, Ausfällen mit Wasser, Fällen als Salz, Umkristallisieren oder durch präparative Säulenchromatographie. Die letztere Methode ist besonders dann von Bedeutung, wenn sich nach Verfahrensvariante a) der Substituent R₂ unter den gegebenen Reaktionsbedingungen wie eine Abgangsgruppe verhält, wodurch stellungsisomere Endprodukte entstehen.

Die bei der Umsetzung nach a) bis f) erhaltenen Verbindungen der Formel I sind Basen und können auf übliche Weise mit anorganischen oder organischen Säuren in ihre pharmazeutisch verwendbaren Salze übergeführt werden. Die Salzbildung kann beispielsweise durchgeführt werden, indem man eine Verbindung der Formel I in einem geeigneten Lösungsmittel, wie beispielsweise in Wasser, Aceton oder Acetonitril, in Alkoholen wie Methanol, Ethanol, Hexanol, Decanol oder in Mischungen dieser Alkohole mit Ethern, bevorzugt mit Diethylether löst, eine mindestens äquivalente Menge der gewünschten Säure zusetzt, für eine gute Durchmischung sorgt und nach beendeter Salzbildung das ausgefallene Salz abfiltriert oder das Lösungsmittel im Vakuum abdestilliert. Gegebenenfalls können die Salze nach der Isolierung umkristallisiert werden.

Pharmazeutisch verwendbare Salze sind beispielsweise Salze mit anorganischen Säuren wie beispielsweise Salzsäure, Bromwasserstoffsäure, Schwefelsäure, Phosphorsäure oder Salpetersäure oder mit organischen Säuren wie Zitronensäure, Weinsäure, Maleinsäure, Fumarsäure, Bernsteinsäure, Äpfelsäure, Methansulfonsäure, Aminosulfonsäure, Essigsäure, Benzoesäure u.ä.

Die als Ausgangsstoffe verwendeten Pyridazone sind bekannt oder können nach an sich bekannten Methoden hergestellt werden. So erhält man:
4,5-Dichlor-3 (2H)-pyridazinon und 4,5-Dichlor-2-methyl-3(2H)-pyridazinon durch Kondensation von Mucochlorsäure mit Hydrazin oder Methylhydrazin nach F. Reichenbacher und K. Drury, DE-PS 10 86 238, weiters 4,5-Dichlor-2-hydroxyethyl-3(2H)-pyridazinon und 4,5-Dichlor-2-diethylaminoethyl-3(2H)-pyridazinon als Analogverbindung zu 4,5-Dichlor-2-dimethyl-aminoethyl-3(2H)-pyridazinon in analoger Reaktion nach R. Schoenbeck und E. Kloimstein, Monatsh. Chem. 99, 15 (1968).

Die als Ausgangsstoffe verwendeten Piperazinylalkylderivate sind bekannt oder können analog zu bekannten Methoden hergestellt werden. So können 4-Aryl- und 4-Heteroaryl-piperazin-Derivate, die in 1-Stellung einen Cyanalkylrest tragen, durch katalytische Hydrierung zu den gewünschten Aminoalkylpiperazinderivaten reduziert werden. Die anzuwendenden präparativen Methoden sind beispielsweise beschrieben in:
Houben-Weyl, "Methoden der organischen Chemie", Bd.XI, 1, Seiten 24 - 108 und 272 - 289, Georg Thieme Verlag Stuttgart (1957), ferner in Jp. Kokai 82 42 679, US-PS 3,398,151, FR-PS 2.261.756 und DE-OS 23 34 009.
Die Pyridazine der Formel IV können nach der in Beispiel 14 angegebenen Methode hergestellt werden.
3,4,6-Dichlorpyridazin wird nach R.H. Mazzoni und P.E. Spoerri, J.Am.Chem.Soc. 76, 2201 (1954) erhalten.

Die neuen Verbindungen der Formel I und ihre pharmazeutisch verwendbaren Salze zeigen in in vitro Modellen eine hervorragende Inhibierung der peripheren Alpha-Rezeptoren (Alpha₁-Adrenozeptoren). Zusätzlich besitzen zahlreiche der untersuchten Substanzen eine gute Wirkung an zentralen 5HT-1A-Rezeptoren.

Aufgrund dieser pharmakologischen Eigenschaften können die neuen Verbindungen allein oder in Mischung mit anderen Wirksubstanzen in Form üblicher galenischer Zubereitungen bei Bluthochdruck und Herzerkrankungen in Medikamenten verwendet werden.

Die Verbindungen der Formel I sind zur Verwendung am Menschen bestimmt und können auf übliche Weise, bei wie beispielsweise oral oder parenteral, verabreicht werden. Vorzugsweise werden sie oral verabreicht, wobei die Tagesdosis etwa 0,015 bis 15 mg/kg Körpergewicht beträgt, vorzugsweise 0,15 bis 1,5 mg/kg Körpergewicht. Bei intravenöser Gabe beträgt die Tagesdosis etwa 1,5 bis 1500 mcg/kg Körpergewicht, bevorzugt etwa 15 - 150 mcg/kg Körpergewicht. Der behandelnde Arzt kann jedoch, abhängig vom Allgemeinzustand und dem Alter des Patienten, der entsprechenden Substanz der Formel I, der Art der Krankheit und der Art der Formulierung, auch Dosen darüber oder darunter verschreiben.

Die Verbindungen der Formel I können allein oder in Verbindung mit anderen pharmazeutisch aktiven Substanzen verabreicht werden, wobei der Gehalt der Verbindungen der Formel I etwa zwischen 0,1 und 99 % liegt. Im allgemeinen liegen die pharmazeutisch aktiven Verbindungen in einer Mischung mit geeigneten inerten Hilfs- und/oder Trägerstoffen oder Verdünnungsmitteln, wie z.B. pharmazeutisch unbedenklichen Lösungsmitteln, Gelatine, Gummi arabicum, Milchzucker, Stärke, Magnesiumstearat, Talk, pflanzlichen Ölen, Polyalkylenglykol, Vaseline und dergleichen vor.
Die pharmazeutischen Präparate können in fester Form, beispielsweise als Tabletten, Dragees, Suppositorien, Kapseln und dergleichen, in halbfester Form, beispielsweise als Salben oder in in flüssiger Form, beispielsweise als Lösungen, Suspensionen oder Emulsionen, vorliegen. Gegebenenfalls sind sie sterilisiert und enthalten Hilfsstoffe, wie Konservierungs-, Stabilisierungs- oder Emulgiermittel, Salze zur Veränderung des osmotischen Druckes und dergleichen.
Insbesondere können pharmazeutische Präparate die erfindungsgemäßen Verbindungen in Kombination mit anderen therapeutisch wertvollen Stoffen enthalten. Mit diesen können mit erfindungsgemäßen Verbindungen beispielsweise zusammen mit den oben angegebenen Hilfs- und/oder Trägerstoffen oder Verdünnungsmitteln zu Kombinationspräparaten formuliert werden.

Die nachstehend als Beispiel angeführten Verbindungen liegen überwiegend in Form ihrer Salze und/oder Solvate vor, wobei die Zahlenangaben das jeweilige stoechiometrische Verhältnis bezeichnen. Im UV-Spektrum bedeutet die erste Zahl die Frequenz, die Zahl in der Klammer (zweite Zahl) die Extinktion.
Verwendete Abkürzungen:
S: Schulter (im UV-Spektrum)
Fp: Schmelzpunkt
% d. Th: % der Theorie
Cl(ges): Chlor (gesamt)
Äqu.: Äquivalent
ber: berechnet
gef: gefunden
Sbl: Sublimation
Umkrist.: Umkristallisation
Umfällg.: Umfällung

### Beispiel 1:

### 2-Methyl-5-brom-4-((2-(4-(2-methoxyphenyl)piperazinyl-1)ethyl)amino)-3(2H)-pyridazinon und 2-Methyl-4-brom-5-((2-(4-(2-methoxyphenyl)piperazinyl-1)ethyl)amino)-3(2H)-pyridazinon

3.0 g (0.0112 mol) 2-Methyl-4,5-dibrom-3(2H)-pyridazinon, 2.64 g (0.0112 mol) 1-(2-Aminoethyl)-4-(2-methoxyphenyl)-piperazin und 1.2 g (0.0112 mol) feingepulvertes Kalium - hydrogencarbonat erwärmt man in 100 ml Dimethylformamid unter gutem Rühren 20 Stunden auf 80 Grad C; saugt sodann heiss vom Anorganischen ab und engt an der Dampfstrahlpumpe ein. Das zurückbleibende braune Öl löst man in 0.5 N HCl, extrahiert 3 mal mit Ether, stellt die Wasserphase alkalisch und verteilt zwischen Wasser und Chloroform. Nach dem Trocknen mit Natriumsulfat und Einengen der Chloroformphase bleiben 4.74 g braunes Öl zurück, das an Silicagel (Matrex Silica Si60, 0.020 - 0.045 mm) durch präparative Säulenchromatographie mit Methylenchlorid-Methanol 40:1.5 aufgetrennt wird. Als erste Fraktion erscheinen 0.67 g 2-Methyl-5-brom-4-((2-(4-(2-methoxyphenyl)piperazinyl-1)ethyl)amino)-3(2H)-pyridazinon, 14.2 % d.Th.; durch Versetzen mit der äquivalenten Menge Fumarsäure in abs. Ethanol erhält man das Fumarat als farblose, kristalline Substanz vom Fp. 185 - 186 Grad C; C 49.5 %, H 5.3 %, Br 15.3 %, N 12.6 %, O 17.3 %; UV in 0.1 N HCl: 208(4.63), 226(S,4.40), 286(S,3.95), 302(4.07).
Durch weiteres Eluieren gewinnt man als 2. Fraktion 2.07 g 2-Methyl-4-brom-5-((2-(4-(2-methoxyphenyl)piperazinyl-1)ethyl)amino)-3(2H)-pyridazinon, das man in in absolutem Ethanol löst und mit Fumarsäure versetzt. Man erhält farbloses kristallines Fumarat (2.3 Äqu.) vom Fp. 125 - 129 Grad C,43.8 % d.Th.; C 46.3 %,H 5.0 %, Br 11.9 %, N 9.9 %, O 26.9 %; UV in 0.1 N HCl: 212(4.63), 226(S,4.40), 282(S,3.83), 302(S,3.71).

### Beispiel 2:

### 2-Methyl-5-chlor-4-((2-(4-(2-methoxyphenyl)piperazinyl-1)ethyl)amino)-3(2H)-pyridazinon und 2-Methyl-4-chlor-5-((2-(4-(2-methoxyphenyl)piperazinyl-1)ethyl)amino)-3(2H)-pyridazinon

10.0 g (0.0559 mol) 2-Methyl-4,5-dichlor-3(2H)-pyridazinon, 13.2 g (0.0559 mol) 1-(2-Aminoethyl)-4-(2-methoxyphenyl)-piperazin und 5.6 g (0.0559 mol) Kaliumhydrogencarbonat werden in 200 ml Acetonitril unter Rühren 20 Stunden unter Rückfluss erhitzt, heiss vom Anorganischen abgesaugt und kaltgestellt. Es fallen 7.7 g 2-Methyl-5-chlor-4-((2-(4-(2-methoxyphenyl) piperazinyl-1)ethyl)amino)-3(2H)-pyridazinon , 36.5% d.Th., als farbloser, kristalliner Niederschlag aus, der nach Umkristalisation aus Ethanol 6.8 g (32,2 %) reine Base liefert. Durch Behandeln mit etherischer HCl in Ethanol verwandelt man in das Dihydrochlorid, Fp.210-220 Grad C; C 45.9 %, H 5.7 %, Cl(ges) 23.6 %, Cl- 16.0 %;, farblose, kristalline Substanz; UV in 0.1 N HCl: 210(4.55), 230(4.30), 300(4.17). Durch Abkühlen der Acetonitril-Mutterlauge gewinnt man eine weisse kristalline Fällung von 4.5 g 2-Methyl-4-chlor-5-((2-(4-(2-methoxy phenyl)piperazinyl-1)ethyl)amino)-3(2H)-pyridazinon, 21.3 % d.Th., welche durch Lösen in Isopropanol und versetzen mit etherischer Salzsäure in das Dihydrochlorid vom Fp. Fp.218 -225 Grad C umgewandelt und durch Umkristallisation aus Isopropanol rein erhalten wird, Fp.223-227 Grad C, farblose Kristalle, 14.3 % d.Th.; C 48.0 %, H 5.7 %, Cl(ges) 23.5 %, Cl- 15.7 %, N 15.0 %, O 7.8 %;
UV in Ethanol: 210(4.5), 230(4.57), 286(4.00), 304(S,3.81).

### Beispiel 3

### 2-t-Butyl-4-chlor-5-((2-(3-(3-trifluormethylphenyl)piperazinyl-1)ethyl)amino)-3(2H)-pyridzinon und 2-t-Butyl-5-chlor-4-((2-(3-(3-trifluormethylphenyl)piperazinyl-1)ethyl)amino)-2(2H)-pyridazinon

15.0 g (0.055 mol) 1-Aminoethyl-4-(3-trifluormethylphenyl)piperazin und 15.2 g (0.069 mol) 2-t-Butyl-4,5-Dichlor-3(2H)-pyridazinon werden mit 6.9 g (0.069 mol) feingepulvertem Kaliumhydrogencarbonat in 100 ml Acetonitril unter Feuchtigkeitsausschluss 96 Stunden unter Rückfluss und gutem Rühren zum Sieden erhitzt; man filtriert vom festen Material ab, engt im Vakuum ein, verteilt zwischen Ether und 1N HCl, extrahiert die saure Phase noch 2 mal mit Ether, stellt sodann mit Natronlauge alkalisch und extrahiert erneut 3 mal mit Chloroform, trocknet die organische Phase mit Natriumsulfat und verdampft das Lösungsmittel; der Rückstand wiegt 30.1 g und wird der präparativen Säulenchromatographie an Kieselgel (Matrex Silica Si60, 0.020 - 0.045 mm) mit dem Eluens Methylenchlorid-Methanol 40:1 unterworfen. Man erhält 18.8 g 2-t-Butyl-4-chlor-5-((2-(3-(3-trifluormethylphenyl)piperazinyl-1)ethyl)amino)-3(2H)-pyridazinon als 1. Fraktion; 74.6 % d.Th. Davon werden 3.80 g in 50 ml Aceton gelöst und mit etherischer Salzsäure in 3.55 g leicht wasserlösliches, farblos kristallines Hydrochlorid (2.8 HCl-Äqu.) vom Fp. 124 - 127 Grad C übergeführt; 54.0 % d.Th.; C 42.4 %, H 6.0 %, Cl(ges) 23.0 %, Cl- 16.7 %, F 9.2 %, N 11.9 %, O 7.5 %; UV in Ethanol: 206(4.39), 210(4.4), 216(4.37), 258(4.08), 304(4.12). Als zweite Fraktion eluiert man 8.3 g isomeres 2-t-Butyl-5-chlor-4-((2-(3-(3-trifluormethylphenyl)piperazinyl-1)ethyl)amino)-3(2H)-pyridazinon; 32.9 % d.Th.; 1.50 g dieser Fraktion werden in 50 ml absolutem Ethanol und mit einem Überschuss an etherischer Salzsäure gefällt und geben 1.20 g Dihydrochlorid vom Fp. 187 - 190 Grad C als leicht im Wasser lösliche, farblose kristalline Substanz. 22.6 % d.Th.; C 47.4 %, H 5.5 %, Cl(ges) 20.0 %, Cl- 13.2 %, F 10.3 %, N 13.2 %, O 3.6 %, UV in Ethanol: 212(S,4.39), 232(4.52), 256(4.19), 290(3.97), 304(S,3.86).

### Beispiel 4:

### 2-Methyl-4-chlor-5-((3-(4-(2-methoxyphenyl)piperazinyl-1)propyl)amino)-3(2H)-pyridazinon und 2-Methyl-5-chlor-4-((3-(4-(2-methoxyphenyl)piperazinyl-1)propyl)amino)-3(2H)-pyridazinon

10.0 g (0.040 mol) 1-Aminopropyl-4-(2-methoxyphenyl)-piperazin und 7.9 g (0.044 mol) 2-Methyl-4,5-dichlor-3(2H)-pyridazinon werden zusammen mit 4.4 g (0.044 mol) Kaliumhydrogencarbonat in 100 ml frischdestilliertem Dioxan 10 Stunden auf 80 Grad C erwärmt und sodann 3 Tage bei Raumtemperatur gerührt. Nach dem Abfiltrieren des anorganischen Materials wird im Vakuum eingeengt, der Rückstand in wässriger Salzsäure gelöst und mit Ether mehrmals extrahiert; die Wasserphase stellt man mit Natronlauge alkalisch, schüttelt 3 mal mit Chloroform aus, trocknet mit Natriumsulfat und erhält nach dem Einengen im Vakuum 15.7 g eines Isomerengemisches. Man trennt durch präparative Säulen chromatographie auf Kieselgel (Matrex Silica Si60 0.020 - 0.045 mm) mit Ether-Methanol 40:5 als mobiler Phase. Als 1. Fraktion eluiert man 7.43 g 2-Methyl-4-chlor-5-((3-(4-(2-methoxyphenyl)piperazinyl-1)propyl)amino)-3(2H)-pyridazinon, 47.5 % d.Th. Davon wurden 5.0 g in absolutem Ethanol gelöst und mit ethanolischer Salzsäure versetzt und lieferten 5.6 g Dihydrochlorid vom Fp. 205 - 220 Grad C; C 48.7 %, H 6.5 %, Cl(ges) 22.8, Cl- 15.3 %, N 15.0 %, O 7.0 %, UV in 0.1 N HCl: 210(4.49), 230(4.54), 282(3.93), 302(S,3.85). Bei fortgesetzter Elution isoliert man als 2. Fraktion 5.95 g 2-Methyl-5-chlor-4-((3-(4-(2-methoxyphenyl)piperazinyl-1)propyl)amino)-3(2H)-pyridazinon, 38.1% d.Th. Nach dem Auflösen in absolutem Ethanol und Versetzen mit ethanolischer Salzsäure geben 4.0 g dieses Produkts 3.9 g Dihydrochlorid vom Fp. 226-228 Grad C; 37.1% d.Th.; C 49.0 %, H 6.5 %, Cl(ges) 22.9, Cl-15.3 %, N 14.8 %, O 6.8 %; UV in 0.1 N HCl: 204(4.48), 230(S,4.54), 286(S,3.95), 302(3.85), 312(S,4.04).

### Beispiel 5:

### 2-Methyl-4-chlor-5-((6-(4-(2-methoxyphenyl)piperazinyl-1)hexyl)amino)-3(2H)-pyridazinon und 2-Methyl-5-chlor-4-((6-(4-(2-methoxyphenyl)piperazinyl-1)hexyl)amino)-3(2H)-pyridazinon

5.8 g (0.020 mol) 1-Aminohexyl-4-(2-methoxyphenyl)-piperazin und 4.45 g (0.025 mol) 2-Methyl-4,5-dichlor-2-methyl-3(2H)-pyridazinon werden mit 2.50 g (0.025 mol) feingepulvertem Kaliumhydrogencarbonat in 100 ml absolutem Ethanol unter Feuchtigkeitsausschluss 48 Stunden unter Rückfluss unter gutem Rühren zum Seiden erhitzt; man entfernt den anorganischen Niederschlag durch Filtration , engt das Filtrat im Vakuum ein, säuert mit 1N HCl an, extrahiert die saure wässrige Phase 3 mal mit Ether, stellt dann mit Natronlauge alkalisch und extrahiert erneut 3 mal mit Chloroform, trocknet die organische Phase mit Natriumsulfat und engt das Lösungsmittel im Vakuum ein; der Rückstand von 10.0 g wird der präparativen Säulenchromatographie an Kieselgel (Waters Prep-Pak) mit dem Eluens Methylenchlorid-Methanol-konz. Ammoniak 40:1.5:0.1 unterworfen. Man eluiert zunächst 3.70 g an 2-Methyl-4-chlor-5-((6-(4-(2-piperazinyl-1)hexyl)amino)-3(2H)-pyridazinon als 1. Fraktion; 42.6 % d.Th. Davon werden 2.00 g in 50 ml Ethanol p.A. gelöst und mit etherischer Salzsäure in 2.20 g wasserlösliches, farbloses Dihydrochlorid vom Fp. 160 -175 Grad C umgewandelt; 38.0 % d.Th.; C 50.3 %, H 6.8 %, Cl(ges) 19.4, Cl- 13.1 %, N 13.2 %, O 10.3 %; UV in Ethanol: 212(4.46), 216(4.45,S), 234(4.50), 286(3.96), 304(3.87,S). Als zweite Fraktion eluiert man 4.1 g isomeres 2-Methyl-5-chlor-4-((6-(4-(2-methoxyphenyl)piperazinyl-1)hexyl)amino)-3(2H)-pyridazinon; 47.2 % d.Th.. Von dieser Fraktion werden 2.00 g in 50 ml p.A. Ethanol gelöst und mit etherischer Salzsäure in 1.50 g wasserlösliches, farbloses kristallines Dihydrochlorid vom Fp. 153 - 165 Grad C umgewandelt; 19.7 % d.Th.; C 52.3 %, H 6.8 %, Cl(ges) 20.6, Cl- 13.8 %, N 13.9 %, O 6.4 %; UV in Ethanol: 212(4.47), 216(4.44), 240(4.29), 302(4.14), 312(3.89,S).

### Beispiel 6:

### 2-Methyl-4-chlor-5-((4-(4-(2-methoxyphenyl)piperazinyl-1)butyl)amino)-3(2H)-pyridazinon und 2-Methyl-5-chlor-4-((4-(4-(2-methoxyphenyl)piperazinyl-1)butyl)amino)-3(2H)-pyridazinon

10.0 g (0.038 mol) 4-Aminobutyl-2-methoxyphenyl-piperazin und 8.5 g (0.048 mol) 2-Methyl-4,5-dichlor-3(2H)-pyridazinon werden zusammen mit 4.75g (0.048 mol) Kaliumhydrogencarbonat in 70 ml wasserfreiem Dimethylsulfoxid gelöst und 15 Stunden bei 80 Grad C gehalten; man verdünnt mit 200 ml Wasser und zieht mehrmals mit Chloroform aus. Die organische Phase wird mit Wasser 3 mal nachgewaschen, anschliessend mit 1 N HCl extrahiert. Man stell die wässerige Phase alkalisch, schüttelt mit Chloroform aus, trocknet mit Natriumsulfat und erhält nach dem Einengen im Vakuum 16.9 g eines Produktgemisches. Die weitere Auftrennung erfolgt durch präparative Säulenchromatographie auf Kieselgel (Matrex Silica Si60 0.020 - 0.045 mm) mit Ether-Methanol 40:5 als mobiler Phase. Als 1. Fraktion isoliert man 5.50 g (35.7 % d.Th.) 2-Methyl-5-chlor-4-((4-(4-(2-methoxyphenyl)piperazinyl-1)butyl)amino)-3(2H)-pyridazinon, 30.1 % d.Th.; es wird in absolutem Ethanol gelöst und mit ethanolischer Salzsäure versetzt und liefert das Dihydrochlorid vom Fp. 205 - 207 Grad C; C 50.1 %, H 6.5 %, Cl(ges) 21.5, Cl- 14.5 %, N 14.4 %, O 7.0 %; UV in Ethanol: 206(4.43), 210(4.50), 244(4.15), 296(4.12), 312(4.09). Nach weiterer Elution erscheint als 2.Fraktion 8.40 g 2-Methyl-4-chlor-5-((4-(4-(2-methoxyphenyl)piperazinyl-1)amino)-3(2H)-pyridazinon, 54.6 % d.Th., das nach dem Auflösen in absolutem Ethanol und Versetzen mit ethanolischer Salzsäure farblos kristallines Dihydrochlorid vom Fp. 183 - 192 Grad C ergibt; C 50.10%, H 6.1 %, Cl(ges) 21.8, Cl- 14.9 %, N 14.9 %, O 7.0%; UV in Ethanol: 210(4.41), 218(4.42), 232(4.46), 236(4.45), 286(3.96).

### Beispiel 7:

### 2-Methyl-4-chlor-5-((2-(4-(2,6-dimethylphenyl)piperazinyl-1)ethyl)amino)-3(2H)-pyridazinon und 2-Methyl-5-chlor-4-((2-(4-(2,6-dimethylphenyl)piperazinyl-1)ethyl)amino)-3(2H)-pyridazinon

9.2 (0.039 mol) 1-Aminoethyl-4-(2,6-dimethylphenyl)-piperazin und 8.8 g (0.049 mol) 2-Methyl-4,5-dichlor-3(2H)-pyridazinon werden zusammen mit 4.9 g (0.049 mol) feingepulvertem Kaliumhydrogencarbonat in 100 ml Toluol unter Ausschluß von Feuchtigkeit 20 Stunden unter Rückfluss und gutem Rühren zum Sieden erhitzt; man filtriert vom anorganischen Material ab, engt im Vakuum ein, löst den Rückstand in 1 N HCl, extrahiert 3 mal mit Ether, stellt sodann die wässerige Phase alkalisch, extrahiert erneut 3 mal mit Chloroform, trocknet die organische Phase mit Natriumsulfat und verdampft das Lösungsmittel; der Rückstand von 15.7 g wird der präparativen Säulenchromatographie an Kieselgel (Waters PrepPak) mit dem Eluens Methylenchlorid-Methanol 40:1 unterworfen. Man erhält 5.70 g an 2-Methyl-4-chlor-5-((2-(4-(2,6-dimethylphenyl)piperazinyl-1)ethyl)amino)-3(2H)-pyridazinon, 32.6 % d.Th., als 1. Fraktion; davon werden 3.80 g in 50 ml absolutem Ethanol gelöst, mit etherischer Salzsäure versetzt und in 3.00 g leicht wasserlösliches, farbloses kristallines Dihydrochlorid vom Fp. 235 - 242 Grad C übergeführt; 32.6 % d.Th.; C 50.7 %, H 6.3 %, Cl(ges) 23.2, Cl- 15.4 %, N 15.6 %, O 4.2 %, UV in Ethanol: 220(4.40), 232(4.49), 290(3.85), 311(3.81).
Als zweite Säulenfraktion erhält man 7.40 g 2-Methyl-5-chlor-4-((2-(4-(2,6-dimethylphenyl)piperzinyl-1)ethyl)amino)-3(2H)-pyridazinon, 42.3 % d.Th.; 4.0 g werden in 50 ml absolutem Ethanol gelöst, mit einem Überschuss an etherischer Salzsäure gefällt und geben 2.40 g leicht wasserlösliches farbloskristallines Hydrochlorid vom Fp. 225 - 232 Grad C; 24.9 % d.Th.; C 55.5 %, H 6.8 % Cl(ges) 17.3, Cl- 6.6 %, N 17.2 %, O 3.2 %; UV in Ethanol: 212(4.35), 216(4.34), 233(4.11), 304(4.09), 312(4.03).

Analog zu den angegebenen Beispielen 1 - 7 werden die folgenden Verbindungen hergestellt:
5-Chlor-4-((2-(4-methoxyphenyl)piperazinyl-1)ethyl)amino)-3(2H)-pyridazinon
Salz: 2.75 HCl ; Solvat: 1.25 H2O
Fp.: 251 - 256 Grad C,
Umkrist.: Ethanol
Ausbeute: 76.2 % d.Th.
C : ber.: 41.95, gef.: 41.8
H : ber.: 5.64, gef.: 5.2
Cl : ber.: 27.32, gef.: 26.6
Cl-: ber.: 20.03, geg.: 19.9
N : ber.: 14.39, gef.: 14.2
O : ber.: 9.68, gef.: 9.0
UV: Solvens: Ethanol,
214 (4.39),302 (3.91),312 (3.85)
2-Methyl-5-chlor-4-((2-(4-phenylpiperazinyl-1)ethyl)amino)-3(2H)-pyridazinon
Salz: 1.6 HCl; Solvat: 0.1 H2O
Fp.: 218 - 220 Grad C, Umkrist.: Ethanol
Ausbeute: 23.5 % d.Th.
C : ber.: 50.05, gef.: 50.0
H : ber.: 5.88, gef.: 6.1
Cl : ber.: 22.59, gef.: 22.5
Cl-: ber.: 13.9, gef.: 14.1
N : ber.: 17.17, gef.: 17.2
O : ber.: 4.31, gef.: 4.2
UV: Solvens: 0.1N HCl,
204 (4.48),232 (4.24),300 (4.11)
2-Methyl-5-chlor-4-(methyl-(2-(2-methoxyphenyl)piperazinyl -1)ethyl)amino)-3(2H)-pyridazinon
Salz: 2.0 HCl; Solvat: 0.5 H2O
Fp.: 224 - 231 Grad C, Umkrist.: Ethanol
Ausbeute: 41.1 % d.Th.
C : ber.: 48.16, gef.: 48.6
H : ber.: 6.17, gef.: 6.1
Cl : ber.: 22.45, gef.: 22.3
Cl-: ber.: 14.96, gef.: 15.1
N : ber.: 14.78, gef.: 14.6
O : ber.: 8.44, gef.: 8.4
UV: Solvens:0.1N HCl,
210 (4.36),218 (4.36),236 (4.37),280 (S,3.95),300 (4.03)
2-Methyl-5-chlor-4-((2-(4-(2-methoxy-5-methylphenyl)piperazinyl-1)ethyl)amino)-3(2H)-pyridazinon
Salz: 1.5 Fumarat;
Fp.: 140 - 144 Grad C, Umkrist.: Aceton
Ausbeute: 25.5 %
C : ber.: 53.05, gef.: 53.5
H : ber.: 5.70, gef.: 6.0
Cl : ber.: 6.26, gef.: 6.5
N : ber.: 12.37, gef.: 12.6
O : ber.: 22.61, gef.: 22.4
UV: Solvens: 0.1N HCl,
206 (4.44),226 (4.27),298 (4.08),310 (S,4.02)
2-Methyl-5-chlor-4-((2-(4-(2-methoxy-4-methylphenyl)piperazinyl-1)ethyl)amino)-3(2H)-pyridazinon
Salz: 1.5 Fumarat;
Fp.: 151 - 154 Grad C, Umkrist.: Aceton
Ausbeute: 27.6 % d.Th.
C : ber.: 53.05, gef.: 53.0
H : ber.: 5.70, gef.: 5.9
Cl : ber.: 6.26, gef.: 5.8
N : ber.: 12.37, gef.: 12.2
O : ber.: 22.61, gef.: 23.1
UV: Solvens: 0.1N HCl,
204 (4.51)226 (S,4.22),300 (4.47)
2-Methyl-5-chlor-4-((2-(4-(3-methoxyphenyl)piperazinyl-1)ethyl)amino)-3(2H)-pyridazinon
Salz: 2.0 HCl;
Fp.: 161 - 169 Grad C, chromatographisch gereinigt
Ausbeute: 31.5 % d.Th.
C : ber.: 47.96, gef.: 47.9
H : ber.: 5.80, gef.: 5.9
Cl : ber.: 23.59, gef.: 23.5
Cl-: ber.: 15.73, gef.: 15.5
N : ber.: 15.54, gef.: 15.5
O : ber.: 7.19, gef.: 7.2
UV: Solvens: Ethanol,
214 (4.48),248 (S,4.06),304 (4.18),312 (S,4.1)
2-Methyl-5-chlor-4-((2-(4-(2-benzyloxyphenyl)piperazinyl-1)ethyl)amino)-3(2H)-pyridazinon
Salz: 2.0 HCl; Solvat: 1.0 H2O
Fp.: 126 - 139 Grad C,
Ausbeute: 51.5 % d.Th. (roh), 19. % d.Th. (rein)
C : ber.: 53.20, gef.: 53.4
H : ber.: 5.39, gef.; 5.7
Cl : ber.: 19.63, gef.: 19.2
Cl-: ber.: 13.09, gef.: 12.8
N : ber.: 12.92, gef.: 12.7
O : ber.: 8.89, gef.: 9.0
UV: Solvens: 1N HCl,
210 (4.58),234 (S,4.18),300 (4.08),311 (S,3.99)
2-Methyl-4-chlor-5-((2-(4-(2-hydroxyphenyl)piperazinyl-1)ethyl)amino)-3(2H)-pyridazinon
Salz: 2.0 HBr; Solvat: 1.5 H2O
Fp.: 208 - 213. ,
Ausbeute: 17.6 % d.Th.
C : ber.: 37.06, gef.: 36.9
H : ber.: 4.94, gef.: 4.5
Cl : ber.: 6.44, gef.: 6.0
N : ber.: 12.71, gef.: 12.6
O : ber.: 10.16, gef.: 10.6
Br : ber.: 29.01, gef.: 29.4
UV: Solvens: 1N HCl,
206 (4.55),230 (S,4.17),235 (S,4.10),300 (4.10),311 (S,4.02)
2-Methyl-5-chlor-4-((2-(4-(2-methylphenyl)piperazinyl-1)ethyl)amino)-3(2H)-pyridazinon
Salz: 1.0 Fumarat; Solvat: 0.5 H2O
Fp.: 185 - 187 Grad C, Umkrist.: Ethanol
Ausbeute: 23.5 % d.Th.
C : ber.: 54.26, gef.: 53.9
H : ber.: 6.00, gef,: 6.0
Cl : ber.: 7.28, gef.: 7.7
N : ber.: 14.38, gef.: 14.3
O : ber.: 18.07, gef.: 18.1
UV: Solvens: 0.1N HCl,
208 (4.43),230 (4.20),300 (4.10),312 (S,4.00)
2-Methyl-5-chlor-4-((2-(4-(3-trifluormethylphenyl)piperazinyl-1)ethyl)amino-3(2H)-pyridazinon
Salz: 1.15 HCl; Solvat: 0.5 H2O
Fp.: 175 - 197 Grad C, Umkrist.: Ethanol
Ausbeute: 26.8 % d.Th.
C : ber.: 46.32, gef.: 46.7
H : ber.: 5.00, gef.: 5.0
Cl : ber.: 16.33, gef.: 16.5
Cl-: ber.: 8.73, gef.: 8.5
N : ber.: 15.00, gef.: 15.1
O : ber.: 5.14, gef.: 5.1
F : ber.: 12.21, gef.: 11.6
UV: Solvens: Ethanol,
208 (4.49),219 (S,4.37),238 (4.06),256 (4.16),304 (4.17)
2-Methyl-5-chlor-4-((2-(4-(4-chlor-3-trifluormethylphenyl)piperazinyl-1)ethyl)amino)-3(2H)-pyridazinon
Salz: 2.0 HCl; Solvat: 0.2 H2O
Fp.: 185 - 188 Grad C, Umfällg.: Ethanol,Diethyl-ether
Ausbeute: 25.6 % d.Th. (roh), 22.1 % d.Th. (rein)
C : ber.: 41.04, gef.: 41.6
H : ber.: 4.29, gef.: 4.3
Cl : ber.: 26.92, gef.: 26.8
Cl-: ber.: 13.5, gef.: 13.5
N : ber.: 13.29, gef.: 13.3
O : ber.: 3.64, gef.: 3.7
F : ber.: 10.82, gef.: 10.3
2-Methyl-5-chlor-4-((2-(4-(3-chlorphenyl)piperazinyl)ethyl)amino)-3(2H)-pyridazinon
Salz: 2.0 HCl; Solvat: 0.15 H2O
Fp.: 195 Grad C (Sbl), chromatographisch gereinigt
Ausbeute: 32.9 d.Th.
C : ber.: 44.59, gef.: 44.7
H : ber.: 5.13, gef.: 5.2
Cl : ber.: 30.97, gef.: 30.6
Cl-: ber.: 15.48, gef.: 15.4
N : ber.: 15.29, gef.: 15.5
O : ber.: 5.15, gef.: 5.1
UV: Solvens: Ethanol,
210 (4.39),216 (4.40),235 (S,4.04),258 (4.13),304 (4.15)
2-Methyl-4-chlor-5-((2-(4-(3,5-dichlorphenyl)piperazinyl-1)ethyl)amino)-3(2H)-pyridazinon
Salz: 1.0 HCl;
Fp.: 191 - 201 Grad C, Umfällg.: Ethanol, Diethyl-ether
Ausbeute: 42.6 % d.Th.
C : ber.: 45.05, gef.: 45.3
H : ber.: 4.67, gef.: 4.7
Cl : ber.: 31.29, gef.: 31.0
Cl-: ber.: 7.82, gef.: 7.8
N : ber.: 15.45, gef.: 15.3
O : ber.: 3.53, gef.: 3.2
2-Methyl-5-chlor-4-((2-(4-(2-fluorophenyl)piperazinyl)-1)ethyl)amino-3(2H)-pyridazinon
Salz: 1 HBr ; Solvat:
Fp.: 210 - 214 Grad C, Umkrist.: Ethanol
Ausbeute: 34.1 % d.Th.
Salz: 1 HBr ; Solvat:
C : ber.: 45.70, gef.: 45.9
H : ber.: 4.96, gef.: 4.8
Cl : ber.: 7.94, gef.: 8.1
N : ber.: 15.68, gef.: 15.5
O : ber.: 3.58, gef.: 3.6
F : ber.: 4.25, gef.: 3.9
Br-: ber.: 17.89, gef.: 18.2
UV: Solvens: 0.1N HCl,
208 (4.24),230 (4.28),300 (4.08),312 (S,3.98)
2-Methyl-5-chlor-4-((2-(4-(4-fluorphenyl)piperazinyl-1)ethyl)amino)-3(2H)-pyridazinon
Salz: 2.0 HCl; Solvat: 0.2 H2O
Fp.: 189 - 195 Grad C, Umkrist.: Ethanol
Ausbeute: 15.8 % d.Th.
C : ber.: 46.16, gef.: 46.6
H : ber.: 5.33, gef.: 5.3
Cl : ber.: 24.04, gef.: 24.0
Cl-: ber.: 16.03, gef.: 16.2
N : ber.: 15.83, gef.: 15.8
O : ber.: 4.34, gef.: 4.6
F : ber.: 4.29, gef.: 4.6
UV: Solvens: Ethanol,
206 (4.32),240 (4.27),304 (4.12),312 (S,4.08),
2-Methyl-5-chlor-4-((2-(4-(4-nitrophenyl)piperazinyl-1)ethyl)amino)-3(2H)-pyridazinon
Salz: 0.9 HCl;
Fp.: 237 - 240 Grad C; Umkrist.: Ethanol
Ausbeute: 17.9 % d.Th.
C : ber.: 47.97, gef.: 48.2
H : ber.: 5.19, gef.: 5.2
Cl : ber.: 15.82, gef.: 16.0
Cl-: ber.: 7.50, gef.: 7.6
N : ber.: 19.74, gef.: 19.8
O : ber.: 11.28, gef.: 10.8
UV: Solvens, Ethanol,
204 (4.34), 232 (4.18),304 (4.09),312 (S,4.06),382 (4.20)
2-t-Butyl-5-chlor-4-((2-(4-(2-methoxyphenyl)piperazinyl-1)ethyl)amino)-3(2H)-pyridazinon
Salz: 1.5 Fumarat;
Fp.: 162 - 165 Grad C, Umfällg..: MeOH,Aceton
Ausbeute: 14. % d.Th.
C : ber.: 54.59, gef.: 54.5
H : ber.: 6.11, gef.: 6.3
Cl : ber.: 5.97, gef.: 6.1
N : ber.: 11.79, gef.: 11.7
O : ber.: 21.55, gef.: 21.3
UV: Solvens: 0.1N HCl,
208 (4.65),282 (S,3.94),300 (4.01)
2-(2-Dimethylaminoethyl)-5-chlor-4-(2-(4-(2-methoxyphenyl)piperazinyl-1)ethyl)amino)-3(2H)-pyridazinon
Salz: 2.95 HBr; Solvat: 3.0 H2O
Fp.: 168 - 178 Grad C, Umkrist.: Ethanol
Ausbeute: 26.1 % d.Th.
C : ber.: 34.65, gef.: 34.8
H : ber.: 5.53, gef.: 5.3
N : ber.: 11.55, gef.: 11.5
O : ber.: 10.99, gef.: 10.9
Br-: ber.: 32.39, gef.: 32.5
UV: Solvens: 0.1N HCl,
206 (4.42),230 (4.12),285 (3.79),302 (4.04),312 (3.86)
2-Hydroxyethyl-5-chlor-4-((2-(4-(2-methoxyphenyl)piperazinyl-1)ethyl)amino)-3(2H)-pyridazinon
Salz: 2.0 HCl; Solvat: 0.45 H2O
Fp.: 171 - 181 Grad C, Umkrist.: Aceton
Ausbeute: 25.6 % d.Th.
C : ber.: 46.68, gef.: 46.4
H : ber.: 5.96, gef.: 5.8
Cl : ber.: 21.75, gef.: 21.7
Cl-: ber.: 14.50, gef.: 14.5
N : ber.: 14.32, gef.: 14.1
O : ber.: 11.29, gef.: 11.0
UV: Solvens:0.1N HCl,
208 (4.52),232 (S,4.14),304 (4.10)
2-(2-Hydroxethyl)-5-chlor-4-((2-(4-(3-trifluormethyl)piperazinyl-1)ethyl)amino)-3(2H)-pyridazinon
Salz: 2.0 HCl; Solvat: 0.85 H2O
Fp.: 113 - 120 Grad C; Umkrist.: Aceton
Ausbeute: 6.9 % d.Th.
C : ber.: 42.73, gef.: 43.3
H : ber.: 5.04, gef.: 5.0
Cl : ber.: 19.91, gef.: 19.4
Cl-: ber.: 13.28, gef.: 12.9
N : ber.: 13.11, gef.: 13.1
O : ber.: 8.39, gef.: 8.5
F : ber.: 10.67, gef.: 10.7
UV: Solvens: Ethanol,
206 (4.41),240 (4.09),258 (4.17),304 (4.18),312 (S,4.11)
2-Methyl-5-chlor-4-((2-(4-(pyridyl-2)piperazinyl-1)ethyl)amino)-3(2H)-pyridazinon
Salz: 2.0 HCl; Solvat: 0.45 H2O
Fp.: 200 - 210 Grad C, Umkrist.: Ethanol
Ausbeute: 19.5 % d.Th.
C : ber.: 44.71, gef.: 44.6
H : ber.: 5.60, gef.: 5.5
Cl : ber.: 24.74, gef.: 24.9
Cl-: ber.: 16.49, gef.: 16.7
N : ber.: 19.55, gef.: 19.6
O : ber.: 5.40, gef.: 5.4
2-Methyl-5-chlor-4-(methyl-((3-(4-(2-methoxyphenyl)piperazinyl-1)propyl)amino)-3(2H)-pyridazinon
Salz: 1.0 Fumarat: Solvat: 1.0 H2O
Fp.: 159 - 165 Grad C, Umkrist.: Ethanol
Ausbeute: 40.8 % d.Th.
C: ber.: 52.98, gef.: 52.8
H : ber.: 7.04, gef.: 6.4
Cl : ber.: 6.52, gef.: 6.4
N : ber.: 12.87, gef.: 13
O : ber.: 20.58, gef.: 21.5
2-Methyl-5-chlor-4-((3-(4-(2-hydroxy-4-methylphenyl)piperazinyl-1)propyl)amino)-3(2H)-pyridazinon
Salz: 1.0 Fumarat;
Fp.: 193 - 197 Grad C, Umkrist.: Ethanol
Ausbeute: 34.3 % d.Th.
C : ber.: 54.38, gef.: 54.5
H : ber.: 5.95, gef.: 6.1
Cl : ber.: 7., gef.: 7.2
N : ber.: 13.79, gef.: 13.7
O : ber.: 18.9, gef.: 18.5
2-Methyl-5-chlor-4-((3-(4-(2-ethoxy-4-methylphenyl)piperazinyl-1)propyl)amino)-3(2H)-pyridazinon
Salz: 2.0 HCl;
Fp.: 220 - 223 Grad C,
Ausbeute: 39. % d.Th.
H : ber.: 6.54, gef.: 6.5
Cl : ber.: 21.58, gef.: 21.4
Cl-: ber.: 14.39, gef.: 14.4
N : ber.: 14.21, gef.: 14.4
O : ber.: 6.49, gef.: 6.9
UV: Solvens: 0.1N HCl,
206 (4.45),226 (S,4.16),302 (4.10),312 (S,4.02),
2-Methyl-5-chlor-4-((3-(4-(2-methylphenyl)piperazinyl-1)piperazinyl-1)propyl)amino)-3(2H)-pyridazinon
Salz: 1.0 Fumarat;
Fp.: 184 - 186 Grad C,
Ausbeute: 30. % d.Th.
C : ber.: 56.15, gef.: 55.6
H : ber.: 6.15, gef.: 6.2
Cl : ber.: 7.21, gef.: 7.3
N : ber.: 14.24, gef.: 14.4
O : ber.: 16.26, gef.: 16.5
UV: Solvens: 0.1N HCl,
206 (4.42),230 (4.14),302 (4.08),312 (S,3.99)
2-Methyl-5-chlor-4-((3-(4-(2-fluorophenyl)piperazinyl-1)propyl)amino)-2(2H)-pyridazinon
Salz: 1.0 Fumarat;
Fp.: 161 - 163 Grad C, Umkrist.: Ethanol
Ausbeute: 28.3 % d.Th.
C : ber.: 53.28, gef.: 53.3
H : ber.: 5.49, gef.: 5.5
Cl : ber.: 7.15, gef.: 7.1
N : ber.: 14.12, gef.: 13.8
O : ber.: 16.13, gef.: 16.6
F : ber.: 3.83, gef.: 3.7
UV: Solvens: 0.1N HCl,
204 (4.4),230 (4.24),302 (4.11),313 (S,4.17)
2-Methyl-5-chlor-4-((3-(4-(4-fluorphenyl))piperazinyl-1)propyl)amino)-3(2H)-pyridazinon
Salz: 2.0 HCl ; Solvat: 3.0 H2O
Fp.: 216 Grad C (Sbl), Umkrist.: Ethanol
Ausbeute: 22.5 % d.Th.
C : ber.: 42.66, gef.: 42.2
H : ber.: 6.17, gef.: 6.0
Cl : ber.: 20.98, gef.: 21.3
Cl-: ber.: 13.99, gef.: 14.0
N : ber.: 13.82, gef.: 14.2
O : ber.: 12.63, gef.: 12.8
F : ber.: 3.75, gef.: 3.5
2-Methyl-5-chlor-4-((2-(4-(pyridyl-2)piperazinyl-1)ethyl)amino)-3(2H)-pyridazinon
Salz: 2.0 HCl; Solvat: 0.15 H2O
Fp.: 219 - 226 Grad C, Umkrist.: Ethanol
Ausbeute: 14.4 % d.Th.
C : ber.: 46.57, gef.: 46.5
H : ber.: 5.87, gef.: 5.8
Cl : ber.: 24.26, gef.: 24.3
Cl-: ber.: 16.17, gef.: 16.2
N : ber.: 19.17, gef.: 19.2
O : ber.: 4.20, gef.: 4.2
UV: Solvens: Ethanol,
206 (4.24),250 (4.26),304 (4.24)
4-Chlor-5-((2-(4-(3-trifluormethyl)piperazinyl-1)ethyl)amino)-3(2H)-pyridazinon
Salz: 2.0 HCl; Solvat: 2.0 H2O
Fp.: 173 - 176 Grad C; Umkrist.: Ethanol
Ausbeute: 60.3 % d.Th.
C : ber.: 39.98, gef.: 40.3
H : ber.: 4.93, gef.: 4.2
Cl : ber.: 20.82, gef.: 21
Cl-: ber.: 13.88, gef.: 14.1
N : ber.: 13.71, gef.: 13.9
O : ber.: 9.40, gef.: 9.5
F : ber.: 11.16, gef.: 11.1
2-Methyl-4-chlor-5-((2-(4-phenylpiperazinyl-1)ethyl)amino)-3(2H)-pyridazinon
Salz: 1.85 HCl; Solvat: 0.7 H2O
Fp.: 171 - 180 Grad C, Umkrist.: Ethanol
Ausbeute: 39. % d.Th.
C : ber.: 47.72, gef.: 48.2
H : ber.: 6.00, gef.: 6.3
Cl : ber.: 23.61, gef.: 23.2
Cl-: ber.: 15.33, gef.: 15
N : ber.: 16.37, gef.: 16.1
O : ber.: 6.36, gef.: 6.2
UV: Solvens: 0.1N HCl,
206 (4.41),230 (4.57),288 (3.92)
2-Methyl-4-chlor-5-((2-(4-(2-methoxy-4-methylphenyl)piperazinyl-1)ethyl)amino)-3(2H)-pyridazinon
Salz: 1.75 Fumarat;
Fp.: 103 - 105 Grad C, Umkrist.: Aceton
Ausbeute: 37.6 % d.Th.
C : ber.: 52.48, gef.: 52.3
H : ber.: 5.59, gef.: 6.1
Cl : ber.: 5.96, gef.: 5.9
N : ber.: 11.77, gef.: 12
O : ber.: 24.20, gef.: 23.7
UV: Solvens: 0.1N HCl,
210 (4.49),228 (4.57),282 (3.99),304 (S,3.81)
2-Methyl-4-chlor-5-((2-(4-(2-methoxy-5-methylphenyl)piperazinyl-1)ethyl)amino)-3(2H)-pyridazinon
Salz: 1.25 Fumarat;
Fp.: 80 - 83 Grad C, Umkrist.: Aceton
Ausbeute: 27.3 %
C : ber.: 53.68, gef.: 53.6
H : ber.: 5.82, gef.: 6.3
Cl : ber.: 6.60, gef.: 6.6
N : ber.: 13.04, gef.: 13.4
O : ber.: 20.85, gef.: 20.1
UV: Solvens: 0.1N HCl,
212 (4.52),228 (4.58),286 (4.03),304 (S,3.80)
2-Methyl-4-chlor-5-((ethyl-(2-(4-(2-methoxyphenyl)piperazinyl-1)ethyl)amino)-3(2H)-pyridazinon
Salz: 2.0 HCl ;
Fp.: 178 - 183 Grad C, Umkrist.: Ethanol
Ausbeute: 31.9 % d.Th. (roh), 22.5 % d.Th.(rein)
C : ber.: 50.17, gef.: 50.4
H : ber.: 6.32, gef.: 6.3
Cl : ber.: 22.21, gef.: 22
Cl-: ber.: 14.81, gef.: 14.7
N : ber.: 14.63, gef.: 14.8
O : ber.: 6.68, gef.: 6.5
2-Methyl-4-chlor-5-((2-(4-(3-methoxyphenyl)piperazinyl-1)ethyl)amino)-3(2H)-pyridazinon
Salz: 2.0 HCl; Solvat: 0.2 H2O
Fp.: 170 - 174 Grad C, Umfällg.: Ethanol,Diethyl-ether
Ausbeute: 47. % d.Th.
C : ber.: 47.58, gef.: 47.6
H : ber.: 5.86, gef.: 6.1
Cl : ber.: 23.41, gef.: 22.9
Cl-: ber.: 15.6, gef.: 15.1
N : ber.: 15.41, gef.: 15.2
O : ber.: 7.75, gef.: 7.6
UV: Solvens: Ethanol
214 (4.6),232 (4.56),250 (S,4.09),290 (3.97),304 (S,3.89)
2-Methyl-4-chlor-5-((2-(4-(2-ethoxyphenyl)piperazinyl-1)ethyl)amino)-3(2H)-pyridazinon
Salz: 1.0 Fumarat;
Fp.: 197 - 199 Grad C, Umkrist.: Aceton
Ausbeute: 35.2 % d.Th.
C: ber.: 54.38, gef.: 54.2
H : ber.: 5.95, gef.: 6
Cl : ber.: 6.98, gef.: 6.9
N : ber.: 13.79, gef.: 13.6
O : ber.: 18.90, gef.: 19.3
2-Methyl-4-chlor-5-((2-(4-(2-hydroxyphenyl)piperazinyl-1)ethyl)amino)-3(2H)-pyridazinon
Salz: 2.25 HBr; Solvat: 1.33 H2O
Fp.: 191 - 195 Grad C,
Ausbeute: 22.6 % d.Th.
C : ber.: 35.82, gef.: 35.9
H : ber.: 4.76, gef.: 4.6
Cl : ber.: 6.22, gef.: 6.1
N : ber.: 12.29, gef.: 11.8
O : ber.: 9.36, gef.: 11.6
Br-: ber.: 31.54, gef.: 30.0
UV: Solvens: 1N HCl,
208 (4.54),230 (4.53),282 (3.96),302 (S,3.8)
2-Methyl-4-chlor-5-((4-(2-hydroxy-4-methylphenyl)piperazinyl-1)ethyl)amino)-3(2H)-pyridazinon
Salz: 2.75 HCl; Solvat: 3.25 H2O
Fp.: 157 - 167 Grad C, Umkrist.: Ethanol
Ausbeute: 78.9 % d.Th.
C : ber.: 40.28, gef.: 40.8
H : ber.: 6.24, gef.: 5.9
Cl : ber.: 24.77, gef.: 24.5
Cl-: ber.: 18.17, gef.: 17.9
N : ber.: 13.05, gef.: 12.8
O : ber.: 15.65, gef.: 16.0
UV: Solvens: Ethanol,
214 (4.80),230 (4.88),286 (4.30),305 (S,4.09)
2-Methyl-4-chlor-5-((2-(4-(2-benzyloxyphenyl)piperazinyl-1)ethyl)amino-3(2H)-pyridazinon
Salz: 1.8 HCl; Solvat: 1.5 H2O
Fp.: 154 - 159 Grad C, Umkrist.: Ethanol
Ausbeute: 49.1 % d.Th. (roh), 25.2 % d.Th. (rein)
C : ber.: 46.22, gef.: 46.2
H : ber.: 4.82, gef.: 5.3
Cl : ber.: 5.68, gef.: 5.1
N : ber.: 11.23, gef.: 10.6
O : ber.: 8.98, gef.: 8.9
Br-: 1.8 ber.: 23.06, gef.: 23.9
UV: Solvens: 0.1N HCl,
208 (4.67),230 (4.54),284 (3.96),304 (S,3.82)
2-Methyl-4-chlor-5-((2-(4-(2-methylphenyl)piperazinyl-1)ethyl)amino)-3(2H)-pyridazinon
Salz: 2.0 Fumarat; Solvat: 2.75 H2O
Fp.: 149 - 155 Grad C, Umkrist.: Ethanol
Ausbeute: 46.3 % d.Th.
C : ber.: 48.52, gef.: 48.7
H : ber.: 5.87, gef.: 5.6
Cl : ber.: 5.51, gef.: 5.6
N : ber.: 10.88, gef.: 10.7
O : ber.: 29.21, gef.: 29.4
UV: Solvens: 0.1N HCl,
200 (4.29),210 (4.45),230 (4.53),286 (3.89),302 (S,3.59)
2-Methyl-4-chlor-5-((2-(4-(3-trifluormethylphenyl)piperazinyl-1)ethyl)amino-3(2H)-pyridazinon
Salz: 3.0 HCl; Solvat: 2.25 H2O
Fp.: 120 - 126 Grad C, Umfällg.: Ethanol, Diethyl-ether
Ausbeute: 38.4 % d.Th.
C : ber.: 38.33, gef.: 38.7
H : ber.: 5.05, gef.: 4.6
Cl : ber.: 25.15, gef.: 25.4
Cl-: ber.: 18.89, gef.: 19.1
N : ber.: 12.42, gef.: 12.4
O : ber.: 8.93, gef.: 9.3
F : ber.: 10.07, gef.: 9.6
UV: Solvens: Ethanol,
208 (4.44),232 (4.51),256 (4.20),294 (3.95),304 (S,3.91)
2-Methyl-5-chlor-4-((2-(4-(3,5-dichlorphenyl)piperazinyl-1)ethyl)amino)-3(2H)-pyridazinon
Salz: 0.75 HCl; Solvat: 0.35 H2O
Fp.: 208 - 221 Grad C, Umfällg.: Ethanol,Diethyl-ether
Ausbeute: 50.3 % d.Th.
C : ber.: 45.34, gef.: 45.2
H : ber.: 4.800, gef.: 4.7
Cl : ber.: 29.52, gef.: 30.0
Cl-: ber.: 5.90, gef.: 6.3
N : ber.: 15.55, gef.: 15.3
O : ber.: 4.80, gef.: 4.7
2-Methyl-4-chlor-5-((2-(4-(3-chlorphenyl)piperazinyl)ethyl)amino)-3(2H)-pyridazinon
Salz: 1.25 HCl; Solvat: 0.4 H2O
Fp.: 211 - 219 Grad C, chromatographisch gereinigt
Ausbeute: 32.9 % d.Th.
C : ber.: 46.93, gef.: 47.2
H : ber.: 5.23, gef.: 5.3
Cl : ber.: 26.48, gef.: 26.0
Cl-: ber.: 10.19, gef.: 10.1
N : ber.: 16.10, gef.: 16.3
O : ber.: 10.19, gef.: 10.1
2-Methyl-4-chlor-5-((2-(4-(2-fluorophenyl)piperazinyl-1)ethyl)amino)-2(2H)-pyridazinon
Salz: 1.0 HBr; Solvat: 0.65 H2O
Fp.: 240 - 243 Grad C, Umkrist.: Ethanol
Ausbeute: 42.4 % d.Th.
C : ber.: 44.54, gef.: 44.6
H : ber.: 5.12, gef.: 5.2
Cl : ber.: 7.73, gef.: 7.5
N : ber.: 15.28, gef.: 15.0
O : ber.: 5.76, gef.: 5.6
F : ber.: 4.14, gef.: 3.6
Br-: ber.: 17.43, gef.: 17.5
UV: Solvens: 0.1N HCl,
212 (S,4.30),230 (4.56),288 (3.89),304, (S,3.78)
2-Methyl-4-chlor-5-((2-(4-(4-fluorphenyl)piperazinyl-1)ethyl)amino)-3(2H)-pyridazinon
Salz: 2.0 HCl; Solvat: 2.25 H2O
Fp.: 155 - 161 Grad C, Umkrist.: Ethanol
Ausbeute: 18.7 % d.Th.
C : ber.: 42.60, gef.: 42.9
H : ber.: 5.78, gef.: 5.3
Cl : ber.: 22.19, gef.: 22.4
Cl-: ber.: 14.79, gef.: 14.7
N : ber.: 14.61, gef.: 14.8
O : ber.: 10.85, gef.: 11.0
F : ber.: 3.96, gef.: 3.6
UV: Solvens: Ethanol,
204 (4.34),208 (4.34),232 (4.54),292 (3.93),304 (S,3.89)
2-Methyl-4-chlor-5-((2-(4-(4-nitrophenyl)piperazinyl-1)ethyl)amino)-3(2H)-pyridazinon
Salz: 0.7 HCl; Solvat: 0.1 H2O
Fp.: 242 - 251 Grad C; Umkrist.: Ethanol
Ausbeute: 10.7 % d.Th.
C : ber.: 48.60, gef.: 48.8
H : ber.: 5.25, gef.: 5.4
Cl : ber.: 14.34, gef.: 14.3
Cl-: ber.: 5.91, gef.: 5.5
N : ber.: 20.00, gef.: 19.8
O : ber.: 11.90, gef.: 11.7
UV: Solvens: Ethanol
208 (4.28),232 (4.47),296 (3.81),312 (3.83),382 (4.13)
2-Methyl-4-chlor-5-((2-(4-(pyridyl-2)piperazinyl-1)ethyl)amino)-3(2H)-pyridazinon
Salz: 2.0 HCl; Solvat; 0.35 H2O
Fp.: 222 - 229 Grad C, Umkrist.: Ethanol
Ausbeute: 36.6 % d.Th.
C : ber.: 44.89, gef.: 45.1
H : ber.: 5.58, gef.: 5.5
Cl : ber.: 24.85, gef.: 24.8
Cl-: ber.: 16.56, gef.: 16.7
N : ber.: 19.63, gef.: 19.6
O : ber.: 5.05, gef.: 5.0
2-t-Butyl-4-chlor-5-((2-(4-(2-methoxyphenyl)piperazinyl-1)ethyl)amino)-3(2H)-pyridazinon
Salz: 1.5 Fumarat; Solvat: 1.2 H2O
Fp.: 220 - 224 Grad C,
Ausbeute: 66.5 % d.Th.
C : ber.: 52.67, gef.: 52.5
H : ber.: 6.29, gef.: 6.3
Cl : ber.: 5.76, gef.: 5.9
N : ber.: 11.38, gef.: 11.4
O : ber.: 23.91, gef.: 23.9
UV: Solvens, 0.1N HCl,
212 (4.68),230 (4.64),282 (4.02),312 (S,3.68)
2-(Dimethylaminoethyl)-4-chlor-5-((2-(4-(2-methoxyphenyl)piperazinyl-1)ethyl)amino)-3(2H)-pyridazinon
Salz: 2.0 Fumarat; Solvat: 2.5 H2O
Fp.: 110 - 115 Grad C,
Ausbeute: 52.2 % d.Th.
C : ber.: 48.91, gef.: 49.1
H : ber.: 6.23, gef.: 5.9
Cl : ber.: 4.88, gef.: 5.0
N : ber.: 11.80, gef.: 11.8
O : ber.: 28.08, gef.: 28.2
UV: Solvens: 0.1N HCl,
210 (4.49),232 (4.57),282 (3.95)309 (S,3.81)
2-Hydroxyethl-4-chlor-5-((2-(4-(2-methoxyphenyl)piperazinyl-1-)ethyl)amino-3(2H)-pyridazinon
Salz: 2.9 HCl; Solvat: 2.7 H2O
Fp.: 130 - 141 Grad C, Umkrist.: Aceton
Ausbeute: 47.3 % d.Th.
C : ber.: 40.17, gef.: 40.6
H : ber.: 6.09, gef.: 5.8
Cl : ber.: 24.34, gef.: 24.4
Cl-: ber.: 18.10, gef.: 18.3
N : ber.: 12.33, gef.: 12.6
O : ber.: 16.05, gef.: 16.4
UV: Solvens: Ethanol,
212 (4.58),232 (4.54),286 (4.00),304 (S,3.85)
2-Hydroxyethyl-4-chlor-5-((2-(4-(3-trifluormethylphenyl)piperazinyl-1)ethyl)amino)-3(2H)-pyridazinon
Salz: 2.75 HCl; Solvat: 2.0 H2O
Fp.: 117 - 121 Grad C, Umkrist.: Ethanol
Ausbeute: 29.1 % d.Th.
C : ber.: 39.20, gef.: 39.2
H : ber.: 5.15, gef.: 4.5
Cl : ber.: 22.84, gef.: 23.2
Cl-: ber.: 16.75, gef.: 16.6
N : ber.: 12.03, gef.: 12.0
O : ber.: 10.99, gef.: 11.3
F : ber.: 9.79, gef.: 9.8
UV: Solvens: Ethanol,
206 (4.41),234 (4.48),256 (4.19),294 (3.99),304 (S,3.95)
2-Phenyl-4-chlor-5-((2-(4-(2-methoxyphenyl)piperazinyl-1)ethyl)amino)-3(2H)-pyridazinon
Salz: 1.0 HBr; Solvat: 0.5 Ethanol . 1 H2O
Fp.: 140 - 147 Grad C, Umkrist.: Ethanol
Ausbeute: 33.5 % d.Th.
C : ber.: 51.30, gef.: 51.7
H : ber.: 5.74, gef.: 5.8
Cl : ber.: 6.31, gef.: 6.2
N : ber.: 12.46, gef.: 12.2
O : ber.: 9.97, gef.: 10.1
Br-: ber.: 14.22, gef.: 14.0
2-Methyl-4-chlor-5-(methyl-((3-(4-(2-methoxyphenyl)piperazinyl-1)propyl)amino)-3(2H)-pyridazinon
Salz: 1.0 Fumarat; Solvat: 0.5 H2O
Fp.: 169 - 173 Grad C; Umkrist.: Ethanol
Ausbeute: 6.1 % d.Th.
C : ber.: 54.28, gef.: 54.0
H : ber.: 6.26, gef.: 6.2
Cl : ber.: 6.68, gef.: 6.6
N : ber.: 13.19, gef.: 13.5
O : ber.: 19.58, gef.: 19.3
2-Methyl-4-chlor-5-((3-(4-(2-hydroxy-4-methylphenyl)piperazinyl-1)propyl)amino)-3(2H)-pyridazinon
Salz: 1.0 Fumarat; Solvat: 0.5 H2O
Fp.: 175 - 181 Grad C, Umkrist.: Ethanol
Ausbeute: 69.1 % d.Th.
C : ber.: 53.44, gef.: 52.7
H : ber.: 6.04, gef.: 6.3
Cl : ber.: 6.86, gef.: 6.9
N : ber.: 13.55, gef.: 13.7
O : ber.: 20.12, gef.: 20.4
2-Methyl-4-chlor-5-((3-(4-(2-ethoxy-4-methylphenyl)piperazinyl-1)propyl)yamino)-3(2H)-pyridazinon
Salz: 2.4 HCl; Solvat: 2.2 H2O
Fp.: 196 - 203 Grad C;
Ausbeute: 48.1 % d.Th.
C : ber.: 46.1, gef.: 45.8
H : ber.: 6.78, gef.: 6.5
Cl: ber.: 22.03, gef.: 22.5
Cl-: ber.: 15.55, gef.: 15.3
N : ber.: 12.80, gef.: 12.9
O : ber.: 12.28, gef.: 12.3
2-Methyl-4-chlor-5-((3-(4-(2-methylphenyl)piperazinyl-1)propyl)amino)-3(2H)-pyridazinon
Salz: 1.0 Fumarat; Solvat: 0.5 H2O
Fp.: 176 - 184 Grad C;
Ausbeute: 40. % d.Th.
C : ber.: 55.14, gef.: 54.7
H : ber.: 6.24, gef.: 6.2
Cl : ber.: 7.08, gef.: 7.3
N : ber.: 13.98, gef.: 14.1
O : ber.: 17.56, gef.: 17.7
UV: Solvens: 0.1N HCl,
208 (4.47),232 (4.56),290 (3.88),302 (S,3.84)
2-Methyl-4-chlor-5-((3-(4-(2-fluorophenyl)piperazinyl-1)propyl)amino)-2(2H)-pyridazinon
Salz: 1.0 Fumarat; Solvat: 1.0 H2O
Fp.: 176 - 179 Grad C, Umfällg..: Ethanol,Aceton
Ausbeute: 42.1 % d.Th.
C : ber.: 51.41, gef.: 51.2
H : ber.: 5.69, gef.: 5.4
Cl : ber.: 6.90, gef.: 7.1
N : ber.: 13.63, gef.: 13.7
O : ber.: 18.68, gef.: 18.8
F : ber.: 3.70, gef.: 3.8
UV: Solvens: 0.1N HCl,
204 (4.39),232 (4.59),290 (3.89),305 (S,3.86)
2-Methyl-4-chlor-5-((3-(4-(4-fluorophenyl)piperazinyl-1)propyl)amino)-3(2H)-pyridazinon
Salz: 3.0 HCl; Solvat: 1.5 H2O
Fp.: 132 - 139 Grad C, Umkrist.: Aceton
Ausbeute: 34.7 % d.Th.
C : ber.: 41.88, gef.: 42.2
H : ber.: 5.47, gef.: 5.6
Cl : ber.: 27.47, gef.: 27.2
Cl-: ber.: 20.6, gef.: 20.8
N : ber.: 13.57, gef.: 13.7
O : ber.: 7.75, gef.: 7.9
F : ber.: 3.68, gef.: 3.4
2-Methyl-4-chlor-5-((3-(4-(pyridyl-2)piperazinyl-1)propyl)amino)-3(2H)-pyridazinon
Salz: 2.0 HCl; Solvat: 0.35 H2O
Fp.: 200 - 214 Grad C; Umkrist.: Ethanol
Ausbeute: 42.1 % d.Th.
C : ber.: 46.19, gef.: 45.9
H : ber.: 5.86, gef.: 5.7
Cl : ber.: 24.06, gef.: 24.4
Cl-: ber.: 16.04, gef.: 16.3
N : ber.: 19.01, gef.: 19.1
O : ber.: 4.89, gef.: 4.9
2-Methyl-4-chlor-5-((6-(4-(2-methoxyphenyl)piperazinyl-1)hexyl)amino)-3(2H)-pyridazinon
Salz: 2.0 HCl; Solvat: 1.5 H2O
Fp.: 160 - 175 Grad C; Umkrist.: Ethanol
Ausbeute: 38.0. % d.Th.
C : ber.: 49.4, gef.: 50.3
H : ber.: 7.16, gef.: 6.8
Cl : ber.: 19.88, gef.: 19.4
Cl-: ber.: 13.25, gef.: 13.1
N : ber.: 13.09, gef.: 13.2
O : ber.: 10.47, gef.: 10.3
UV: Solvens: Ethanol,
212 (4.46),216 (S,4.45),234 (4.50),286 (3.96),304 (S,3.87)

### Beispiel 8:

### 2-Methyl-5-((2-(4-(2-methoxyphenyl)piperazinyl-1)ethyl)amino)-3(2H)-pyridazinon

3.0 g (0.00794 mol) 4-Chlor-2-methyl-5-((2-(4-(2-methoxyphenyl)piperazinyl-1)ethyl)amino)-3(2H)-pyridazinon löst man in 10 ml absolutem Ethanol, gibt 1.38 g (0.01 mol) Kaliumcarbonat und 0.3 g Pd/C (10%-ig) zu und hydriert bei Raumtemperatur bis zum Stillstand der Wasserstoffaufnahme. Nach dem Abfiltrieren des Katalysators und des anorganischen Materials engt man im Vakuum ein und erhält 2.5 g 2-Methyl-5-((2-(4-(2-methoxyphenyl)piperazinyl-1)ethyl)amino)-3(2H)-pyridazinon (91.7 % d.Th.) als farblosen, kristallinen Rückstand, der in Isopropanol heiss gelöst und mit etherischer Salzsäure versetzt wird. Es fallen 2.0 g (66.3 % d.Th.) Hydrochlorid vom Fp. 237-245 Grad C an; C 55.6 %, H 7.0 %, Cl(ges) 9.3, Cl- 9.3 %, N 18.3 %, O 9.8 %.

### Beispiel 9:

### 4-((2-(4-(2-Methoxyphenyl)piperazinyl-1)ethyl)amino)-3(2H)-pyridazinon

1.66 g (0.00312 mol) 6-Chlor-4-((2-(4-(2-methoxyphenyl) piperazinyl-1)ethyl)amino)-pyridazinon werden in 100 ml Ethanol mit 0.0046 Mol NaOH und 100 mg 10%-iger Palladium-Kohle bei 60 Grad C 1 Stunde bis zum Stillstand der Wasserstoffaufnahme hydriert; man filtriert vom Katalysator ab, engt ein und zieht mit heissem absolutem Alkohol aus, der sodann mit alkoholischer Salzsäure angesäuert wird. Es fallen 1.28 g 2-Methyl-4-((2-(4-(2-methoxyphenyl)piperazinyl-1)ethyl)amino)-3(2H)-pyridazinon (81.5% d.Th.) als farblos-kristallines Hydrochlorid vom Fp. 235-244 Grad C (unter Zersetzung) an; C 40.3 %, H 6.4 %, Cl-24.3 %, N 13.9 %, O 15.0 %.

In analoger Weise werden hergestellt:
4-((2-(4-(2-Hydroxyphenyl)piperazinyl-1)ethyl)amino)-3(2H)-pyridazinon
Salz: 1.0 HBr;
Fp.: 253 - 260 Grad C, Umkrist.: Ethanol
Ausbeute: 73.3 % d.Th.
C : ber.: 48.49, gef.: 48.5
H : ber.: 5.60, gef.: 5.8
N : ber.: 17.67, gef.: 17.5
O : ber.: 8.07, gef.: 8.4
Br : ber.: 20.16, gef.: 20.1
2-Methyl-4-((2-(4-phenylpiperazinyl-1)ethyl)amino)-3(2H) pyridazinon
Salz: 3.2 HCl; Solvat: 2.6 H2O
Fp.: 225 - 230 Grad C, Umkrist.: Ethanol
Ausbeute: 59.7 % d.Th.
C : ber.: 42.81, gef.: 43.1
H : ber.: 6.64, gef.: 5.7
Cl-: ber.: 23.79, gef.: 24.0
N : ber.: 14.68, gef.: 14.9
O : ber.: 12.08, gef.: 12.3
UV: Solvens: Ethanol,
206 (4.33),252 (4.08),298 (4.16),310 (S,3.97)
2-Methyl-4-((2-(4-(2-methoxyphenyl)piperazinyl-1)ethyl)amino)-3(2H)-pyridazinon
Salz: 2.0 HCl;
Fp.: 130 - 139 Grad C, Umkrist.: Ethanol
Ausbeute: 66. % d.Th.
C : ber.: 51.93, gef.: 51.7
H : ber.: 6.54, gef.: 6.6
Cl-: ber.: 17.03, gef.: 17.1
N : ber.: 16.82, gef.: 16.7
O : ber.: 7.69, gef.: 7.9
UV: Solvens: 1N HCl,
200 (3.81),204 (3.88),220 (4.14),282 (S,4.05),296 (4.12)
2-Methyl-4-(ethyl-(2-(4-(2-methoxyphenyl)piperazinyl-1)ethyl)amino)-3(2H)-pyridazinon
Salz: 1 Fumarat ; Solvat: 0.33 H2O
Fp.: 140 - 144 Grad C, Umfällg.: Ethanol,Diethyl-ether
Ausbeute: 46.3 % d.Th.
C : ber.: 58.41, gef.: 58.3
H : ber.: 6.86, gef.: 7.0
N : ber.: 14.19, gef.: 14.1
O : ber.: 20.53, gef.: 20.5
2-Methyl-4-((2-(4-(2-methoxy-5-methylphenyl)piperazinyl-1)ethyl)amino)-3(2H)-pyridazinon
Salz: 2.0 HCl;
Fp.: 235 Grad C (Sbl), Umkrist.: Ethanol
Ausbeute: 49.4 % d.Th.
C : ber.: 52.58, gef.: 52.2
H : ber.: 6.83, gef.: 7.2
Cl-: ber.: 16.34, gef.: 16.6
N : ber.: 16.14, gef.: 16.0
O : ber.: 8.11, gef.: 8.0
UV: Solvens: 0.1N HCl,
214 (4.57),224 (S,4.55),282 (4.03)
2-Methyl-4-((2-(4-(2-methoxy-4-methylphenyl)piperazinyl-1)ethyl)amino)-3(2H)-pyridazinon
Salz: 1.25 Fumarat ; Solvat: 2 H2O
Fp.: 161 - 164 Grad C, Umkrist.: Aceton
Ausbeute: 70.1 % d.Th.
C : ber.: 53.52, gef.: 53.7
H : ber.: 6.74, gef.: 6.5
N : ber.: 13.00, gef.: 13.0
O : ber.: 26.73, gef.: 26.8
UV: Solvens: 0.1N HCl,
206 (4.35),210 (4.34),290 (4.15)
2-Methyl-4-((6-(4-(2-methoxyphenyl)piperazinyl)hexyl)amino)-3(2H)-pyridazinon
Salz: 3.0 HCl;
Fp.: 225 - 228 Grad C, Umfällg.: Ethanol,Diethyl-ether
Ausbeute: 32.3 % d.Th.
C : ber.: 47.75, gef.: 47.8
H : ber.: 6.23, gef.: 6.2
Cl-: ber.: 23.49, gef.: 23.1
N : ber.: 14.47, gef.: 15.5
O : ber.: 7.07, gef.: 7.4
2-Methyl-4-((2-(4-(2-ethoxyphenyl)piperazinyl-1)ethyl)amino)-3(2H)-pyridazinon
Salz: 2.0 HCl;
Fp.: 196 - 204 Grad C;
Ausbeute: 66.4 % d.Th.
C : ber.: 53.03, gef.: 52.5
H : ber.: 6.79, gef.: 6.9
Cl-: ber.: 16.48, gef.: 16.3
N : ber.: 16.27, gef.: 16.2
O : ber.: 7.45, gef.: 8.0
UV: Solvens: 0.1N HCl,
208 (4.28),227 (S,4.02),288 (4.05
2-Methyl-4-((2-(4-(2-hydroxyphenyl)piperazinyl-1)ethyl)amino)-3(2H)-pyridazinon
Salz: 2.25 HBr; Solvat: 3.2 H2O
Fp.: 188 - 196 Grad C,
Ausbeute: 32.3 % d.Th.
C : ber.: 35.88, gef.: 36.1
H : ber.: 5.61, gef.: 5.1
N : ber.: 12.31, gef.: 12.3
O : ber.: 14.62, gef.: 14.6
Br-: ber.: 31.59, gef.: 31.9
UV: Solvens: 1N HCl,
206 (4.46),225 (S,4.11),288 (4.15),304 (S,4.01)
2-Methyl-4-((2-(4-(2,6-dimethylphenyl)piperazinyl-1)ethyl)amino)-3(2H)-pyridazinon
Salz: 2.0 HCl; Solvat: 0.6 H2O
Fp.: 235 - 240 Grad C, Umkrist.: Ethanol
Ausbeute: 47.7 % d.Th.
C : ber.: 53.67, gef.: 53.7
H : ber.: 7.16, gef.: 7.3
Cl-: ber.: 16.69, gef.: 16.4
N : ber.: 16.47, gef.: 16.5
O : ber.: 6.02, gef.: 6.01
2-Methyl-4-((2-(4-(3-trifluoromethylphenyl)piperazinyl-1)ethyl)amino)-3(2H)-pyridazinon
Salz: 2.2 HCl; Solvat: 3.4 H2O
Fp.: 124 - 133 Grad C; Umkrist.: Ethanol
Ausbeute: 59.8 % d.Th.
C : ber.: 41.35, gef.: 41.8
H : ber.: 5.97, gef.: 5.9
Cl-: ber.: 14.92, gef.: 15.5
N : ber.: 13.40, gef.: 13.4
O : ber.: 13.46, gef.: 14.0
F : ber.: 10.90, gef.: 10.4
UV: Solvens: Ethanol,
204 ( 4.4),258 (4.20),300 (4.20),312 (S,4.05)
2-Methyl-4-((2-(4-(2-fluorophenyl)piperazinyl-1)ethyl)amino)-3(2H)-pyridazinon
Salz: 1.2 HCl;
Fp.: 240 - 248 Grad C,
Ausbeute: 95.5 % d.Th.
C : ber.: 54.43, gef.: 54.4
H : ber.: 6.23, gef.: 6.4
Cl-: ber.: 11.34, gef.: 11.0
N : ber.: 18.67, gef.: 18.5
O : ber.: 4.27, gef.: 4.4
F : ber.: 5.06, gef.: 5.1
2-Methyl-4-((2-(4-(4-fluorophenyl)piperazinyl-1)ethyl)amino) 3(2H)-pyridazinon
Salz: 2.6 HCl; Solvat: 2.6 H2O
Fp.: 236 - 240 Grad C, Umkrist.: Ethanol
Ausbeute: 81.7 % d.Th.
C : ber.: 43.17, gef.: 43.6
H : ber.: 6.35, gef.: 5.7
Cl-: ber.: 19.49, gef.: 19.9
N : ber.: 14.81, gef.: 15.1
O : ber.: 12.18, gef.: 12.3
F : ber.: 4.02, gef.: 3.4
UV: Solvens: Ethanol,
206 (4.28),234 (4.03),244 (4.03),300 (4.18),312 (S,4.06)
2-t-Butyl-4-((2-(4-(2-methoxyphenyl)piperazinyl-1)ethyl)amino)-3(2H)-pyridazinon
Salz: 2.0 HBr; Solvat: 0.1 H2O
Fp.: 238 - 242 Grad C, Umkrist.:
Ausbeute: 86.5 % d.Th.
C : ber.: 45.93, gef.: 46.1
H : ber.: 6.09, gef.: 6.2
N : ber.: 12.75, gef.: 12.5
O : ber.: 6.12, gef.: 6.4
Br-: 2. ber.: 29.47, gef.: 28.8
UV: Solvens: 0.1N HCl,
206 (4.51),225 (S,4.24),290 (4.20),312 (S,3.76)
2-t-Butyl-4-((2-(4-(3-trifluoromethyl)piperazinyl-1)ethyl)amino)-3(2H)-pyridazinon
Salz: 2.0 HCl;
Fp.: 194 - 198 Grad C; Umkrist.: Ethanol
Ausbeute: 48.8 % d.Th.
C : ber.: 50.81, gef.: 50.9
H : ber.: 6.09, gef.: 6.2
Cl-: ber.: 14.28, gef.: 14.1
N : ber.: 14.11, gef.: 14.2
O : ber.: 3.22, gef.: 3.2
F : ber.: 11.48, gef.: 11.4
UV: Solvens: Ethanol,
206 (3.79),260 (4.13),298 (4.10)
2-(2-Dimethylaminoethyl)-4-((2-(4-(2-methoxyphenyl)piperazinyl-1)ethyl)amino)-3(2H)-pyridazinon
Salz: 3.0 HBr; Solvat: 1.5 H2O
Fp.: 231 - 237 Grad C, Umfällg.: Ethanol,Diethyl-ether
Ausbeute: 41.7 % d.Th. (roh), 34.8 % d.Th. (rein)
C : ber.: 37.63, gef.: 37.8
H : ber.: 5.71, gef.: 5.6
N : ber.: 12.54, gef.: 12.4
O : ber.: 8.35, gef.: 8.4
Br-: ber.: 35.76, gef.: 35.8
UV: Solvens: 0.1N HCl,
208 (4.43),229 (S,4.17),285 (S,4.26),296 (4.28),312 (S,4.12)
2-Hydroxyethyl-4-((2-(4-(2-methoxyphenyl)piperazinyl-1)ethyl)amino)-3(2H)-pyridazinon
Salz: 3.15 HCl; Solvat: 3.4 H2O
Fp.: 181 - 190 Grad C, Umkrist.: Ethanol
Ausbeute: 34.1 % d.Th.
C : ber.: 41.53, gef.: 41.6
H : ber.: 6.78, gef.: 6.5
Cl-: ber.: 20.32, gef.: 20.4
N : ber.: 12.74, gef.: 12.8
O : ber.: 18.63, gef.: 18.7
UV: Solvens: Ethanol,
210 (4.44),300 (4.15),312 (S,3.95)
2-(2-Hydroxyethyl)-4-((2-(4-(3-trifluoromethylphenyl)piperazinyl-1)ethyl)amino)-3(2H)-pyridazinon
Salz: 2.4 HCl; Solvat: 1.35 H2O
Fp.: 121 - 129 Grad C; Umkrist.: Aceton
Ausbeute: 84.1 % d.Th.
C : ber.: 43.61, gef.: 44
H : ber.: 5.41, gef.: 5.3
Cl-: ber.: 16.26, gef.: 16.3
N : ber.: 13.38, gef.: 13.6
O : ber.: 10.24, gef.: 10.4
F : ber.: 10.90, gef.: 10.4
UV: Solvens: Ethanol,
206 (4.34),211 (S,4.27),260 (4.21),300 (4.21),312 (S,4.06)
2-Methyl-4-((2-(4-(pyridyl-2)piperazinyl-1)ethyl)amino)-3(2H)-pyridazinon
Salz: 2.4 HCl; Solvat: 0.65 H2O
Fp.: 235 - 237 Grad C, Umkrist.: Ethanol
Ausbeute: 89. % d.Th.
C : ber.: 46.67, gef.: 46.7
H : ber.: 6.29, gef.: 6.2
Cl-: ber.: 20.66, gef.: 20.6
N : ber.: 20.41, gef.: 20.1
O : ber.: 6.41, gef.: 6.4
UV: Solvens: Ethanol,
204 (4.10),252 (4.26),300 (4.26)
4-((3-(4-(2-Methoxyphenyl)piperazinyl-1)propyl)amino)-3(2H)-pyridazinon
Salz: 2.0 HCl; Solvat: 0.1 H2O
Fp.: 245 - 256 Grad C,
Ausbeute: 81.9 % d.Th.
C : ber.: 51.61, gef.: 51.4
H : ber.: 6.55, gef.: 6.6
Cl-: ber.: 17.10, gef.: 17.1
N : ber.: 16.72, gef.: 16.7
O : ber.: 8.02, gef.: 8.0
4-((3-(4-(2-Ethoxyphenyl)piperazinyl-1)propyl)amino)-3(2H)-pyridazinon
Salz: 2.1 HCl;
Fp.: 258 - 269 Grad C,
Ausbeute: 20.3 % d.Th.
C : ber.: 50.99, gef.: 50.9
H : ber.: 6.89, gef.: 6.7
Cl-: ber.: 16.64, gef.: 17.0
N : ber.: 15.65, gef.: 15.7
O : ber.: 9.83, gef.: 9.9
2-Methyl-4-((3-(4-(2-methoxyphenyl)piperazinyl-1)propyl)amino)-3(2H)-pyridazinon
Salz: 2.85 HCl;
Fp.: 238 - 246 Grad C, Umkrist.: Ethanol
Ausbeute: 86.2 % d.Th.
C : ber.: 47.87, gef.: 47.5
H : ber.: 6.67, gef.: 6.8
Cl-: ber.: 21.20, gef.: 21.1
N : ber.: 14.69, gef.: 14.7
O : ber.: 9.57, gef.: 9.8
2-Methyl-4-((3-(4-(2-hydroxy-4-methylphenyl)piperazinyl-1)propyl)amino)-3(2H)-pyridazinon
Salz: 1.0 Fumarat;
Fp.: 176 - 180 Grad C, Umkrist.: Ethanol
Ausbeute: 21.9 % d.Th.
C : ber.: 57.25, gef.: 57.7
H : ber.: 6.48, gef.: 6.8
N : ber.: 14.51, gef.: 14.2
O : ber.: 21.55, gef.: 21.0
2-Methyl-4-((3-(4-(2-ethoxy-4-methylphenyl)piperazinyl-1)propyl)amino)-3(2H)-pyridazinon
Salz: 2.8 HCl; Solvat: 1.75 H2O
Fp.: 202 - 205 Grad C,
Ausbeute: 97.3 % d.Th.
C : ber.: 48.59, gef.: 48.5
H : ber.: 7.24, gef.: 7.1
Cl-: ber.: 19.12, gef.: 19.4
N : ber.: 13.49, gef.: 13.5
O : ber.: , gef.:
UV: Solvens: 0.1N HCl,
206 (4.35),296 (4.15),312 (S,3.92)
2-Methyl-4-((3-(4-(2-fluorophenyl)piperazinyl-1)propyl)amino)-3(2H)-pyridazinon
Salz: 1.1 HCl;
Fp.: 232 - 238 Grad C,
Ausbeute: 97.2 % d.Th.
C : ber.: 56.08, gef.: 56.1
H : ber.: 6.56, gef.: 6.7
Cl-: ber.: 10.12, gef.: 10.2
N : ber.: 18.17, gef.: 18.1
O : ber.: 4.15, gef.: 4.2
F : ber.: 4.93, gef.: 4.7
2-Methyl-4-((3-(4-(4-fluorophenyl)piperazinyl-1)propyl)amino)-3(2H)-pyridazinon
Salz: 2.0 HCl; Solvat: 0.45 H2O
Fp.: 210 - 212 Grad C; Umkrist.: Ethanol
Ausbeute: 84.4 % d.Th.
C : ber.: 50.70, gef.: 50.9
H : ber.: 6.36, gef.: 6.4
Cl : ber.: 16.63, gef.: 16.9
Cl-: ber.: 16.63, gef.: 16.9
N : ber.: 16.42, gef.: 16.9
O : ber.: 5.44, gef.: 5.6
F : ber.: 4.46, gef.: 4.3
2-Methyl-4-((3-(4-(pyridyl-2)piperazinyl-1)propyl)amino)-3(2H)-pyridazinon
Salz: 2.0 HCl; Solvat: 0.6 H2O
Fp.: 214 - 219 Grad C, Umkrist.: Ethanol
Ausbeute: 97.1 % d.Th.
C : ber.: 49.54, gef.: 50.1
H : ber.: 6.65, gef.: 7.0
Cl : ber.: 17.2, gef.: 16.6
Cl-: ber.: 17.2, gef.: 16.6
N : ber.: 20.39, gef.: 19.9
O : ber.: 6.21, gef.: 6.4
4-((4-(4-(2-Methoxyphenyl)piperazinyl-1)butyl)amino)-3(2H)-pyridazinon
Salz: 3.0 HCl; Solvat: 0.38 H2O
Fp.: 170 - 182 Grad C,
Ausbeute: 79.6 % d.Th.
C : ber.: 48.21, gef.: 48.2
H : ber.: 6.54, gef.: 6.4
Cl : ber.: 22.39, gef.: 22.3
Cl-: ber.: 22.39, gef.: 22.3
N : ber.: 14.79, gef.: 14.7
O : ber.: 8.04, gef.: 8.0
2-Methyl-4-((4-(4-(2-methoxyphenyl)piperazinyl-1)butyl)amino)-3(2H)-pyridazinon
Salz: 2.0 HCl; Solvat: 0.25 H2O
Fp.: 193 - 202 Grad C, Umkrist.: Ethanol
Ausbeute: 76.7 % d.Th.
C : ber.: 53.51, gef.: 53.2
H : ber.: 7.07, gef.: 7.3
Cl-: ber.: 15.80, gef.: 15.8
N : ber.: 15.60, gef.: 15.3
O : ber.: 8.02, gef.: 7.8
5-((2-(4-(3-Trifluorophenyl)piperazinyl-1)ethyl)amino)-3(2H)-pyridazinon
Salz: 3.0 HCl; Solvat: 0.3 H2O
Fp.: 197 - 205 Grad C; Umkrist.: Aceton
Ausbeute: 66.9 % d.Th.
C : ber.: 42.35, gef.: 42.7
H : ber.: 4.93, gef.: 4.9
Cl : ber.: 22.06, gef.: 21.5
Cl-: ber.: 22.06, gef.: 21.5
N : ber.: 14.53, gef.: 14.8
O : ber.: 4.31, gef.: 4.5
F : ber.: 11.82, gef.: 11.6
2-Methyl-5-((2-(4-phenylpiperazinyl-1)ethyl)amino)-3(2H)-pyridazinon
Salz: 3.25 HCl; Solvat: 1.15 H2O
Fp.: 251 - 256 Grad C, Umkrist.: Ethanol
Ausbeute: 76.2 % d.Th.
C : ber.: 45.11, gef.: 44.9
H : ber.: 6.36, gef.: 5.8
Cl : ber.: 25.46, gef.: 25.9
Cl-: ber.: 25.46, gef.: 25.9
N : ber.: 15.47, gef.: 15.7
O : ber.: 7.60, gef.: 7.7
UV: Solvens: Ethanol,
204 (S,4.59),208 (4.62),230 (4.66),252 (S,4.23),284 (4.23
2-Methyl-5-((2-(4-(2-methoxyphenyl)piperazinyl-1)ethyl)amino)-3(2H)-pyridazinon
Salz: 1.0 HCl; Solvat: 0.4 H2O
Fp.: 237 - 245 Grad C,
Ausbeute: 91.7 % d.Th.
C : ber.: 55.85, gef.: 55.6
H : ber.: 6.98, gef.: 7
Cl : ber.: 9.16, gef.: 9.3
Cl-: ber.: 9.16, gef.: 9.3
N : ber.: 18.09, gef.: 18.3
O : ber.: 9.92, gef.: 9.8
2-Methyl-5-((2-(4-(2-methoxy-4-methylphenyl)piperazinyl-1)ethyl)amino)-3(2H)-pyridazinon
Salz: 1.5.Fumarat ; Solvat: 0.5 H2O
Fp.: 176 - 178 Grad C, Umkrist.: Aceton
Ausbeute: 84.1 % d.Th.
C : ber.: 55.55, gef.: 55.6
H : ber.: 6.34, gef.: 6.5
N : ber.: 12.96, gef.: 12.8
O : ber.: 25.16, gef.: 25.1
UV: Solvens: 0.1N HCl,
198 (4.34),212 (4.53),224 (4.53),280 (4.01)
2-Methyl-5-((2-(4-(2-methoxy-5-methylphenyl)piperazinyl-1)ethyl)amino)-3(2H)-pyridazinon
Salz: 1.0 Fumarat;
Fp.: 183 - 185 Grad C, Umkrist.: Aceton
Ausbeute: 76.8 % d.Th.
C : ber.: 58.34, gef.: 58.0
H : ber.: 6.60, gef.: 6.8
N : ber.: 14.79, gef.: 14.7
O : ber.: 20.27, gef.: 20.5
UV: Solvens: 0.1N HCl,
210 ( 4.5),288 (4.02),304 (S,3.83)
2-Methyl-5-(ethyl-(2-(4-(2-methoxyphenyl)piperazinyl-1)ethyl)amino)-3(2H)-pyridazinon
Salz: 3.0 HCl; Solvat: 3.0 H2O
Fp.: 125 - 130 Grad C, Umfällg.: Isopropanol,Diethyl-ether
Ausbeute: 62.3 % d.Th.
C : ber.: 44.91, gef.: 44.8
H : ber.: 7.16, gef.: 6.8
Cl : ber.: 19.88, gef.: 19.6
Cl-: ber.: 19.88, gef.: 19.7
N : ber.: 13.09, gef.: 13.1
O : ber.: 14.96, gef.: 15.7
2-Methyl-5-((2-(4-(2-ethoxyphenyl)piperazinyl-1)ethyl)amino)-3(2H)-pyridazinon
Salz: 1.0 Fumarat; Solvat: 0.6 H2O
Fp.: 175 - 178 Grad C,
Ausbeute: 64.8 % d.Th.
C : ber.: 57.04, gef.: 56.8
H : ber.: 6.70, gef.: 6.7
N : ber.: 14.46, gef.: 14.5
O : ber.: 21.80, gef.: 22.0
UV: Solvens: 1N HCl,
208 (4.51),224 (4.51),276 (4.02)
2-Methyl-5-((2-(4-(2-hydroxyphenyl)piperazinyl-1)ethyl)amino)-3(2H)-pyridazinon
Salz: 3.15 HBr; Solvat: 1.3 H2O
Fp.: 190 - 198 Grad C, Umkrist.: Ethanol
Ausbeute: 44.2 % d.Th.
C : ber.: 33.60, gef.: 33.7
H : ber.: 4.77, gef.: 4.8
N : ber.: 11.52, gef.: 11.5
O : ber.: 8.69, gef.: 8.7
Br-: 3.15 ber.: 41.42, gef.: 41.3
UV: Solvens: 1N HCl,
210 (4.63),224 (4.53),280 (4.03)
2-Methyl-4-((2-(4-(2-methylphenyl)piperazinyl-1)ethyl)amino)-3(2H)-pyridazinon
Salz: 1.5 Fumarat;
Fp.: 178 - 182 Grad C, Umkrist.: Ethanol
Ausbeute: 93. % d.Th.
C : ber.: 57.48, gef.: 57.8
H : ber.: 6.23, gef.: 6.2
N : ber.: 13.96, gef.: 13.9
O : ber.: 22.33, gef.: 22.4
2-Methyl-5-((2-(4-(2,6-dimethylphenyl)piperazinyl-1)ethyl)amino)-3(2H)-pyridazinon
Salz: 2.0 HCl; Solvat: 2.1 H2O
Fp.: 260 Grad C (Sbl); Umkrist.: Ethanol
Ausbeute: 73.7 % d.Th.
C : ber.: 50.46, gef.: 51
H : ber.: 7.40, gef.: 7.4
Cl-: ber.: 15.68, gef.: 15.2
N : ber.: 15.49, gef.: 15.1
O : ber.: 10.97, gef.: 11.3
UV: Solvens: Ethanol,
206 (4.26),214 (4.35),224 (4.38),232 (4.38),280 (3.88)
2-Methyl-4-((2-(4-(2-fluorophenyl)piperazinyl-1)ethyl)amino)-3(2H)-pyridazinon
Salz: 1.5 Fumarat;
Fp.: 167 - 169 Grad C, Umkrist.: Ethanol
Ausbeute: 92. % d.Th.
C : ber.: 54.65, gef.: 54.5
H : ber.: 5.58, gef.: 5.8
N : ber.: 13.85, gef.: 13.9
O : ber.: 22.16, gef.: 22.4
F : ber.: 3.76, gef.: 3.3
2-Methyl-5-((2-(4-(4-fluorophenyl)piperazinyl-1)ethyl)amino)-3(2H)-pyridazinon
Salz: 3.0 HCl; Solvat: 0.7 H2O
Fp.: 162 - 168 Grad C ; Umkrist.: Ethanol
Ausbeute: 73.5. % d.Th.
C : ber.: 45.05, gef.: 45.5
H : ber.: 5.87, gef.: 5.7
Cl-: ber.: 23.46, gef.: 22.9
N : ber.: 15.45, gef.: 15.6
O : ber.: 6.00, gef.: 6.3
F : ber.: 4.19, gef.: 4.0
UV: Solvens: Ethanol,
208 (4.40),218 (4.39),230 (4.50),286 (3.94)
2-Methyl-5-((2-(4-(pyridyl-2)piperazinyl-1)ethyl)amino)-3(2H)-pyridazinon
Salz: 3.0 HCl; Solvat: 0.5 H2O
Fp.: 260 Grad C (Sbl); Umkrist.: Ethanol
Ausbeute: 80.9 % d.Th.
C : ber.: 44.40, gef.: 44.4
H : ber.: 6.06, gef.: 6.1
Cl : ber.: 24.58, gef.: 24.4
N : ber.: 19.42, gef.: 19.5
O : ber.: 5.55, gef.: 5.6
2-t-Butyl-5-((2-(4-(2-methoxyphenyl)piperazinyl-1)ethyl)amino)-3(2H)-pyridazinon
Salz: 1.5 Fumarat;
Fp.: 173 - 177 Grad C,
Ausbeute: 79.2 % d.Th.
C : ber.: 57.95, gef.: 57.6
H : ber.: 6.66, gef.: 6.7
N : ber.: 12.51, gef.: 12.4
O : ber.: 22.87, gef.: 23.3
UV: Solvens: 0.1N HCl,
210 (4.48),226 (4.50),276 (4.05)
2-t-Butyl-5-((2-(4-(3-trifluormethyl)piperazinyl-1)ethyl)amino)-3(2H)-pyridazinon
Salz: 3.0 HCl; Solvat: 0.25 H2O
Fp.: 228 - 230 Grad C; Umkrist.: Aceton
Ausbeute: 77.2 % d.Th.
C : ber.: 46.94, gef.: 47.0
H : ber.: 5.91, gef.: 5.9
Cl-: ber.: 19.79, gef.: 19.7
N : ber.: 13.03, gef.: 13.2
O : ber.: 3.72, gef.: 3.8
F : ber.: 10.61, gef.: 10.4
UV: Solvens: Ethanol,
210 (S,4.43),222 (S,4.50),230 (4.53),258 (4.23),278 (S,4.05)
2-(2-Dimethylaminoethyl)-5-((2-(4-(2-methoxyphenyl)piperazinyl-1)ethyl)amino)-3(2H)-pyridazinon
Salz: 2.1 HBr; Solvat: 0.1 H2O
Fp.: 236 - 246 Grad C, Umkrist.: Isopropanol
Ausbeute: 42.4 % d.Th.
C : ber.: 44.08, gef.: 44.1
H : ber.: 6.04, gef.: 6.4
N : ber.: 14.69, gef.: 14.5
O : ber.: 5.87, gef.: 5.9
Br-: 2.1 ber.: 29.32, gef.: 29.1
2-Hydroxyethyl-5-((2-(4-(2-methoxyphenyl)piperazinyl -1)ethyl)amino)-3(2H)-pyridazinon
Salz: 3.0 HCl; Solvat: 0.6 H2O
Fp.: 190 - 200 Grad C, Umkrist.: Ethanol
Ausbeute: 70.2 % d.Th.
C : ber.: 46.23, gef.: 46.4
H : ber.: 6.37, gef.: 6.2
Cl : ber.: 21.55, gef.: 21.2
N : ber.: 14.19, gef.: 14.0
O : ber.: 11.67, gef.: 11.5
2-(2-Hydroxyethyl)-5-((2-(4-(3-trifluormethyl)piperazinyl-1)ethyl)amino)-3(2H)-pyridazinon
Salz: 2.15 HCl; Solvat: 1.05 H2O
Fp.: 190 - 194 Grad C; Umkrist.: Aceton
Ausbeute: 88.2 % d.Th.
C : ber.: 44.86, gef.: 44.9
H : ber.: 5.60, gef.: 5.3
Cl-: ber.: 14.98, gef.: 15.3
N : ber.: 13.77, gef.: 14.0
O : ber.: 9.59, gef.: 9.8
F : ber.: 11.20, gef.: 10.7
UV: Solvens: Ethanol,
212 (4.33),218 (4.31),232 (4.35),258 (4.14),294 (3.91)
2-Phenyl-5-((2-(4-(2-methoxyphenyl)piperazinyl-1)ethyl)amino)-3(2H)-pyridazinon
Salz: 1.0 HBr; Solvat: 0.1 H2O
Fp.: 272 - 276 Grad C,
Ausbeute: 85.4 % d.Th.
C : ber.: 56.58, gef.: 56.8
H : ber.: 5.82, gef.: 5.9
N : ber.: 14.34, gef.: 14.3
O : ber.: 6.88, gef.: 7.3
Br-: ber.: 16.37, gef.: 16.4
2-Methyl-5-((3-(4-(2-methoxyphenyl)piperazinyl-1)propyl)amino)-3(2H)-pyridazinon
Salz: 3.0 HCl; Solvat: 1.45 H2O
Fp.: 241 - 248 Grad C, Umfällg.: Ethanol,Aceton
Ausbeute: 61.7 % d.Th.
C : ber.: 46.29, gef.: 46.1
H : ber.: 6.52, gef.: 6.8
Cl-: ber.: 21.58, gef.: 21.6
N : ber.: 14.21, gef.: 14.3
O : ber.: 11.20, gef.: 11.2
UV: Solvens: Ethanol,
212 (4.56),218 (S,4.51),230 (4.51),284 (4.01)
1-Methyl-5-((3-(4-(2-ethoxy-4-methylphenyl)piperazinyl-1)propyl)amino)-3(2H)-pyridazinon
Salz: 3.25 HCl; Solvat: 3.1 H2O
Fp.: 218 - 227 Grad C,
Ausbeute: 65.4 % d.Th.
C : ber.: 45.05, gef.: 44.9
H : ber.: 7.28, gef.: 7.1
Cl-: ber.: 20.58, gef.: 20.6
N : ber.: 12.51, gef.: 12.6
O : ber.: 14.57, gef.: 14.8
UV: Solvens: 0.1N HCl,
212 (4.49),228 (4.46),280 (3.98)
2-Methyl-4-((3-(4-(2-methylphenyl)piperazinyl-1)propyl)amino)-3(2H)-pyridazinon
Salz: 1.0 Fumarat;
Fp.: 194 - 197 Grad C, Umkrist.: Ethanol
Ausbeute: 94.6 % d.Th.
C : ber.: 60.38, gef.: 60.4
H : ber.: 6.83, gef.: 7.0
N : ber.: 15.31, gef.: 15.1
O : ber.: 17.49, gef.: 17.2
2-Methyl-5-((3-(4-(4-fluorphenyl)piperazinyl-1)propyl)amino)-3(2H)-pyridazinon
Salz: 3.0 HCl; Solvat: 0.9 H2O
Fp.: 176 - 181 Grad C; Umkrist.: Ethanol
Ausbeute: 76.8 % d.Th.
C : ber.: 45.90, gef.: 46.1
H : ber.: 5.97, gef.: 6.3
Cl-: ber.: 22.58, gef.: 22.4
N : ber.: 14.87, gef.: 15.1
O : ber.: 6.03, gef.: 6.6
F : ber.: 4.03 gef.: 3.5
2-Methyl-5-((3-(4-(pyridyl-2)piperazinyl-1)propyl)amino) 3(2H)-pyridazinon
Salz: 3.0 HCl;
Fp.: 232 - 239 Grad C; Umkrist.: Ethanol
Ausbeute: 70.6 % d.Th.
C : ber.: 46.64, gef.: 46.8
H : ber.: 6.22, gef.: 6.3
Cl: ber.: 24.29, gef.: 23.9
Cl-: ber.: 24.29, gef.: 23.9
N : ber.: 19.20, gef.: 19.4
O : ber.: 3.65, gef.: 3.6
2-Methyl-5-((4-(4-(2-methoxyphenyl)piperazinyl-1)butyl)amino)-3(2H)-pyridazinon
Salz: 3.0 HCl; Solvat: 1.0 H2O
Fp.: 168 - 176 Grad C, Umkrist.: Ethanol
Ausbeute: 66.8 % d.Th.
C : ber.: 48.15, gef.: 47.6
H : ber.: 6.87, gef.: 6.8
Cl-: ber.: 21.32, gef.: 21.3
N : ber.: 14.04, gef.: 14.0
O : ber.: 9.62, gef.: 9.4
2-Methyl-5-((6-(4-(2-methoxyphenyl)piperazinyl)hexyl)amino)-3(2H)-pyridazinon
Salz: 3.0 HCl; Solvat: 0.15 H2O
Fp.: 174 - 185 Grad C, Umfällg.: Ethanol,Diethyl-ether
Ausbeute: 79.2 % d.Th.
C : ber.: 51.65, gef.: 51.7
H : ber.: 7.15, gef.: 7.2
Cl-: ber.: 20.79, gef.: 20.4
N : ber.: 13.69, gef.: 13.6
O : ber.: 6.72, gef.: 6.7
→

### Beispiel 10:

### 5-Methoxy-2-methyl-4-((2-(4-(2-methoxyphenyl)piperazinyl-1)ethyl)amino)-3(2H)-pyridazinon

4.2 g (0.011 mol) 5-Chlor-2-methyl-4-((2-(4-(2-methoxyphenyl)piperazinyl-1)ethyl)amino)-3(2H)-pyridazinon werden in Methanol, in dem 0.010 mol Natriummethylat gelöst sind, 50 Stunden unter Rückfluss erhitzt, sodann im Vakuum eingeengt. Der Rückstand wird mit Wasser aufgenommen, wobei 1.6 g 5-Methoxy-2-methyl-4-((2-(4-(2-methoxyphenyl) piperazinyl-1)ethyl)amino)-3(2H)-pyridazinon (38.9% d.Th.) ausfallen. Man saugt ab, trocknet, löst in Aceton und überführt durch Zugabe von Fumarsäure bei Rückflusstemperatur in 1.50 g (24.5% d.Th.) Fumaratsalz, Fp. 144-148 Grad C; C 54.0 %, H 6.1 %, N 12.5 %, O 27.4 %; UV in 0.1 N HCl: 208(4.42), 226(S,4.22), 300.4.47).

### Beispiel 11:

### 5-Chlor-4-((2-(4-(3-trifluormethylphenyl)piperazinyl-1)ethyl)amino)-3(2H)-pyridazinon

4.25 g (0.0093 mol) 2-t-Butyl-5-chlor-4-((2-(4-(3-trifluormethylphenyl)piperazinyl-1)ethyl)amino)-3(2H)-pyridazinon rührt man mit 50 ml konzentrierter wässeriger Salzsäure bei Raumtemperatur 72 Stunden lang, stellt basisch und extrahiert 3 mal mit Chloroform, engt die organische Phase ein, trocknet mit Natriumsulfat und reibt den Rückstand mit Aceton an. Die kristalline Fällung von 5-Chlor-4-((2-(4-(3-trifluormethylphenyl)piperazinyl-1)ethyl)amino)-3(2H)-pyridazinon wiegt 2.40 g (47.7% d.Th.). Man überführt durch Auflösen in 100 ml heissem absoluten Alkohol und Zugabe von etherischer Salzsäure in 2.20 g (46.7% d.Th.) Dihydrochlorid vom Fp. 220 - 223 Grad C; C 40.6 %, H 4.2 %, Cl(ges) 21.1 %, Cl- 14.2 %, F 11.5 %, N 13.9 %, O 8.7 %; UV in 0.1 N HCl: 208(4.42), 226(S,4.22), 300(4.47).

Die folgende Substanz wird in analoger Weise hergestellt:
4-Chlor-5-((2-(4-(2-methoxyphenyl)piperazinyl)ethyl)amino)-3(2H)-pyridazinon
Salz: 1.5 Fumarat; Solvat: 2.0 H2O
Fp.: 211 - 213 Grad C , Umkrist.: Ethanol
Ausbeute: 58.5 % d.Th.
C : ber.: 48.13, gef.: 48.3
H : ber.: 5.62, gef.: 5.4
Cl: ber.: 6.18, gef.: 5.6
N : ber.: 12.20, gef.: 12.4
O : ber.: 27.87, gef.: 28.3
UV: Solvens: 0.1N HCl,
210 (4.38),228 (4.42),280 (3.81),304 (3.72)

### Beispiel 12:

### 2-Methyl-6-chlor-4-((3-(4-(2-methoxyphenyl)piperazinyl-1)propyl)amino)-3(2H)-pyridazinon

6.0 g (0.0159 mol) feinverriebenes 6-Chlor-4-((3-(4-(2-methoxyphenyl)piperazinyl-1)propyl)amino)-3(2H)-pyridazinon werden in 100 ml 2n NaOH suspendiert und bei 60 Grad C mit 1.51 ml Dimethylsulfat (0.0159 mol) 2 Stunden gerührt und dann abgekühlt: man extrahiert mehrmals mit Chloroform, trocknet die organische Phase und engt ein. Aus dem öligen Rückstand kristallisieren über Nacht 3.50 g (56.2% d.Th.) unreines 2-Methyl-6-chlor-4-((3-(4-(2-methoxyphenyl)piperazinyl-1)propyl)amino-3(2H)-pyridazinon, das durch präparative Chromatographie an Kieselgel (Waters PrepPak) gereinigt wird. Die Reinfraktion wird in Isopropanol gelöst und mit etherischer Salzsäure versetzt und gibt 0.85 g (11.2% d.Th.) weisses, kristallines Dihydrochlorid, Fp. 218-229 Grad C; C 40.6 %, H 4.2 %, Cl(ges) 21.1 %, Cl- 14.2 %, F 11.5 %, N 13.9 %, O 8.7 %.

### Beispiel 13:

### 6-Chlor-2-ethyl-4-((3-(4-(2-methoxyphenyl)piperazinyl-1)propyl)amino)-3(2H)-pyridazinon

1.80 g (0.00474 mol) feinverriebenes 6-Chlor-4-((3-(4-(2-methoxyphenyl)piperazinyl-1)propyl)amino)-3(2H)-pyridazinon suspendiert man in 80 ml 2n NaOH, fügt 1.8 ml (0.014 mol) Ethyljodid zu und rührt bei Raumtemperatur 90 Minuten: danach fügt man erneut 1.8 ml Ethyljodid zu und rührt weitere 2 Stunden. Man dampft das Lösungsmittel ab, nimmt in Wasser auf und extrahiert mit Chloroform. Aus der getrockneten organischen Phase bleibt beim Einengen ein braunes Öl zurück, das beim Lösen in Ethanol und Versetzen mit ethanolischer Salzsäure 0.70 g (30.6% d.Th.) reines Dihydrochlorid von 6-Chlor-2-ethyl-4-((3-(4-(2-methoxyphenyl)piperazinyl-1)propyl)amino-3(2H)-pyridazinon, Fp.202-207 Grad C; als weisse kristalline Substanz ergibt; C 49.4 %, H 6.4 %, Cl(ges) 21.8 %, Cl- 14.6 %, N 14.3 %, O 7.7 %.

### Beispiel 14:

### 6-Chlor-4-((4-(4-(2-methoxyphenyl)piperazinyl-1)butyl)amino)-3(2H)-pyridazinon

4.00 g (0.00985 mol) 6-Chlor-3-methoxy-4-((4-(4-(2-methoxyphenyl)piperazinyl-1)propyl)amino)-pyridazin werden in 40 ml Eisessig gelöst und mit 40 ml 63%-iger HBr versetzt. Man kocht 2 Stunden unter Rückfluss, versetzt mit 200 ml Wasser, neutralisiert mit 30%-iger KOH auf pH 6 und saugt die ausgefallene Substanz ab und wäscht gut mit Wasser. Man erhält 3.85 g (99.7% d.Th.)6-Chlor-4-((4-(4-(2-methoxyphenyl)piperazinyl-1)butyl)amino-3(2H)-pyridazinon, reinigt durch Umkristallisieren aus Ethanol unter Zugabe von Kohle, fügt sogleich zur Lösung ethanolische Salzsäure zu und erhält 3.26 g (68.7% d.Th.) reines Dihydrochlorid, Fp. 247-252 Grad C; C 47.2 %, H 6.0 %, Cl(ges) 21.9 %, Cl- 14.6 %, N 14.4 %, O 10.0 %

Die zur Durchführung des angeführten Beispiels erforderlichen Ausgangsverbindungen werden wie nachstehend angeführt hergestellt:

### 6-Chlor-3-methoxy-4-((4-(4-(2-methoxyphenyl)piperazinyl-1)butyl)amino)pyridazin

3.28 g (0.008 mol) 3,6-Dichlor-4-((4-(4-(2-methoxyphenyl)-1-piperazinyl)butyl)amino)pyridazin und 0.32 g (0.008 mol) Natriummethylat werden in 150 ml Methanol 144 Std. bei 50 Grad gerührt. Anschliessend engt man im Vakuum ein, löst den Rückstand in Chloroform und schüttelt mit Wasser aus. Man dampft das Lösungsmittel ab, löst in Ether, filtriert klar und fällt mit etherischer HCl das Hydrochlorid von 6-Chlor-3-methoxy-4-((4-(4-(2-methoxyphenyl)piperazinyl-1)butyl)amino)pyridazin, 3.14 Äqu. HCl) vom Fp.: 139-150 Grad C, Ausbeute: 91.1 % d.Th.

### 3,6-Dichlor-4-((4-(4-(2-methoxyphenyl)piperazinyl-1) butyl)amino)pyridazin

9.25 g (0.050 mol) 3,4,6-Trichlorpyridazin werden mit 6.90 g (0.050 mol) wasserfreiem gepulvertem Kaliumcarbonat und 13.15 g (0.050 mol) 1-(4-Aminobutyl)-4-(2-methoxyphenyl)piperazin in 1350 ml trockenem Acetonitril 96 Stunden. bei Raumtemperatur gerührt. Anschliessend wird abgesaugt und das Filtrat in Vakuum eingeengt. Den Rückstand nimmt man in Ethanol auf und fällt mit etherischer HCl das Trihydrochlorid von 3,6-Dichlor-4-((4-(4-(2-methoxyphenyl)piperazinyl-1)butyl)amino) pyridazin vom Fp.: 155-170 Grad C; Ausbeute: 54.2 % d.Th.

Die nachfolgenden Verbindungen werden in analoger Weise hergestellt:
6-Chlor-4-((2-(4-(2-methoxyphenyl)piperazinyl-1)ethyl)amino)-3(2H)-pyridazinon
Salz: 2.1 HCl; Solvat: 1.16 H2O
Fp.: 241 - 247 Grad C;
Ausbeute: 86.8 % d.Th.
C : ber.: 44.26, gef.: 44.6
H : ber.: 5.77, gef.: 5.3
Cl : ber.: 23.82, gef.: 23.7
Cl-: ber.: 16.14, gef.: 16.1
N : ber.: 15.18, gef.: 15.1
O : ber.: 10.96, gef.: 10.9
2-Methyl-6-chlor-4-((2-(4-(2-methoxyphenyl)piperazinyl-1)ethyl)amino)3(2H)-pyridazinon
Salz: 2.0 HCl ; Solvat: 0.05 H2O
Fp.: 232 - 237 Grad C
Ausbeute: 63.0 % d.Th.
C : ber.: 47.79, gef.: 47.8
H : ber.: 5.82, gef.: 5.8
Cl : ber.: 23.67, gef.: 23.5
Cl-: ber.: 15.83, gef.: 15.8
N : ber.: 15.48, gef.: 15.4
O : ber.: 7.24, gef.: 7.2
6-Chlor-4-((2-(4-(2-iso-propoxyphenyl)piperazinyl-1)ethyl)amino)-3(2H)-pyridazinon
Salz: 1.0 HBr; Solvat: 0.3 H2O
Fp.: 280 - 295 Grad C, Umkrist.: Ethanol
Ausbeute: 12.7 % d.Th.
C : ber.: 47.72, gef.: 48.1
H : ber.: 5.82, gef.: 6.0
Cl : ber.: 7.41, gef.: 6.6
N : ber.: 14.64, gef.: 14.6
O : ber.: 7.70, gef.: 8.0
Br-: ber.: 16.71, gef.: 16.7
UV: Solvens: 1N HCl,.
210 (4.48),234 (S,4.08), 246 (S,3.94),288 (4.13),309 (S,3.82)
6-Chlor-4-((3-(4-(2-methoxyphenyl)piperazinyl-1)propyl)amino)-3(2H)-pyridazinon
Salz: 2.0 HCl; Solvat: 0.35 H2O
Fp.: 267 - 275 Grad C;
Ausbeute: 88.1 % d.Th.
C: ber.: 47.3, gef.: 47.2
H: ber.: 5.89, gef.: 5.8
Cl : ber.: 23.27, gef.: 23.1
Cl-: ber.: 15.51, gef.: 15.5
N: ber.: 15.32, gef.: 15.3
O : ber.: 8.23, gef.: 8.2
6-Chlor-4-((3-(4-(2-methoxy-4-methylphenyl)piperazinyl-1)propyl)amino)-3(2H)-pyridazinon
Salz: 2.0 HBr; Solvat: 3.5 H2O
Fp.: 191 - 195 Grad C, Umkrist.: Ethanol
Ausbeute: 81.3 % d.Th.
C : ber.: 37.08, gef.: 37.3
H : ber.: 5.72, gef.: 5.2
Cl : ber.: 5.75, gef.: 5.5
N : ber.: 11.35, gef.: 11.4
O : ber.: 14.27, gef.: 14.7
Br-: ber.: 25.91, gef.: 25.9
UV: Solvens: 0.1N HCl,
206 ( 4.6), 226 (S,4.19), 288 (4.28), 09 (S,4.03)
2-Methyl-6-chlor-(3-(4-(4-(2-ethoxyphenyl)piperazinyl-1)propyl)amino)-3(2H)-pyridazinon
Salz: 2.0 HCl
Fp.: 211 - 215 Grad C; Ausbeute: 7.9 % d.Th.
C : ber.: 50.17, gef.: 50.3
H : ber.: 6.32, gef.: 6.4
Cl : ber.: 22.21, gef.: 21.9
Cl-: ber.: 14.81, gef.: 14.6
N : ber.: 14.63, gef.: 14.5
O : ber.: 6.68, gef.: 7.1
6-Chlor-2-methyl-4-((3-(4-(2-methoxyphenyl)piperazinyl-1)propyl)amino)-3(2H)-pyridazinon
Salz: 2.0 HCl
Fp.: 218 - 229 Grad C
Ausbeute: 14.5 % d.Th.
C : ber.: 47.62, gef.: 47.1
H : ber.: 6.23, gef.: 6.0
Cl : ber.: 22.19, gef.: 22.7
Cl-: ber.: 14.80, gef.: 15.0
N : ber.: 14.61, gef.: 14.7
O : ber.: 9.35, gef.: 9.5
6-Chlor-2-ethyl-4-((3-(4-(2-methoxyphenyl)piperazinyl-1)propyl)amino)-3(2H)-pyridazinon
Salz: 2.0 HCl; Solvat: 0.35 H2O
Fp.: 202 - 207 Grad C
Ausbeute: 30.6 % d.Th.
C : ber.: 49.51, gef.: 49.4
H : ber.: 6.38, gef.: 6.4
Cl : ber.: 21.92, gef.: 21.8
Cl-: ber.: 14.62, gef.: 14.6
N : ber.: 14.44, gef.: 14.3
O : ber.: 7.75, gef.: 7.7
6-Chlor-2-hydroxyethyl-4-((3-(4-(2-methoxyphenyl)piperazinyl-1)propyl)amino)-3(2H)-pyridazinon
Salz: 2.0 HCl; Solvat: 1.0 H2O
Fp.: 168 - 175 Grad C
Ausbeute: 25.8 % d.Th.
C : ber.: 46.84, gef.: 47.5
H : ber.: 6.29, gef.: 5.8
Cl : ber.: 20.74, gef.: 20.5
Cl-: ber.: 13.83, gef.: 13.9
N : ber.: 13.66, gef.: 13.5

### Beispiel A:

### Bestimmung der Affinität von Verbindungen der Formel I zu Alpha₁-Adrenozeptoren.

Die Affinität von Verbindungen der allgemeinen Formel I zu Alpha₁-Adrenozeptoren wurde nach der von R.S. Williams, D.F. Dukes und R.F. Lefkowitz im J. Cardiovasc. Pharmacol 3, 522 - 531 (1981) beschriebenen Methode bestimmt. Nach dieser Methode mißt man die kompetitive Verdrängung von tritiiertem Prazosin (2-(4-(2-Furoyl)-1-piperazinyl)-4-amino-6,7-dimethoxy-chinazolin) an Rattenherzmembranen durch die Testsubstanzen und ermittelt als IC₅₀ (50 % inhibition concentration) diejenige Konzentration, welche eine 50 %ige Hemmung der spezifischen Bindung des tritiierten Prazosins an die Alpha₁-Adrenozeptoren in Rattenherzmembranen bewirkt.

Aus den IC₅₀-Werten wurden die konzentrationsunabhängigen Inhibitorkonstanten K_{I}-Alpha₁ entsprechend den Angaben von Y. Cheng und H.W. Prusoff in Biochem. Pharmacol. 22, 3099 - 3108 (1973) ermittelt.

Die Ergebnisse dieser Untersuchungen sind in der folgenden Tabelle zusammengefaßt:

### Inhibitionskonstanten am Alpha-1-Adrenoceptor:

2-Methyl-5-brom-4-((2-(4-(2-methoxyphenyl)piperazin-1)ethyl)amino)-3(2H)-pyridazinon
Ki = 1.33
2-Methyl-4-brom-5-((2-(4-(2-methoxyphenyl)piperazinyl-1)ethyl)amino)-3(2H)-pyridazinon
Ki = 5.88
2-Methyl-5-chlor-4-((2-(4-(2-methoxyphenyl)piperazinyl-1)ethyl)amino)-3(2H)-pyridazinonn
Ki = 1.51
2-Methyl-4-chlor-5-((2-(4-(2-methoxyphenyl)piperazinyl-1)ethyl)amino)-3(2H)-pyridazinon
Ki = 7.57
2-Methyl-4-chlor-5-((3-(4-(2-methoxyphenyl)piperazinyl-1)propyl)amino)-3(2H)-pyridazinon
Ki = 32.2
2-Methyl-5-chlor-4-((3-(4-(2-methoxyphenyl)piperazinyl-1)propyl)amino)-3(2H)-pyridazinon
Ki = 3.48
2-Methyl-4-chlor-5-((4-(4-(2-methoxyphenyl)piperazinyl-1)butyl)amino)-3(2H)-pyridazinon
Ki = 6.05
2-Methyl-5-chlor-4-((4-(4-(2-methoxyphenyl)piperazinyl-1)butyl)amino)-3(2H)-pyridazinon
Ki = 2.15
5-Chlor-4-((2-(4-(2-methoxyphenyl)piperazinyl-1)ethyl)amino)-3(2H)-pyridazinon
Ki = 2.95
2-Methyl-5-chlor-4-((2-(4-phenylpiperazinyl-1)ethyl)amino)-3(2H)-pyridazinon
Ki = 86.7
2-Methyl-5-chlor-4-((2-(4-(2-methoxy-5-methylphenyl)piperazinyl-1)ethyl)amino)-3(2H)-pyridazinon
Ki = 6.01
2-Methyl-5-chlor-4-((2-(4-(2-methoxy-4-methylphenyl)piperazinyl-1)ethyl)amino)-3(2H)-pyridazinon
Ki = 22.4
2-Methyl-5-chlor-4-((2-(4-(2-benzyloxyphenyl)piperazinyl-1)ethyl)amino)-3(2H)-pyridazinon
Ki = 2.54
2-Methyl-5-chlor-4-((2-(4-(2-hydroxyphenyl)piperazinyl-1)ethyl)amino)-3(2H)-pyridazinon
Ki = 2.55
2-Methyl-5-chlor-4-((2-(4-(2-methylphenyl)piperazinyl-1)ethyl)amino)-3(2H)-pyridazinon
Ki = 1.21
2-Methyl-5-chlor-4-((2-(4-(3-trifluormethylphenyl)piperazinyl-1)ethyl)amino)-3(2H)-pyridazinon
Ki = 47.3
2-Methyl-5-chlor-4-((2-(4-(2-fluorophenyl)piperazinyl-1)ethyl)amino)-3(2H)-pyridazinon
Ki = 2.51
2-t-Butyl-5-chlor-4-((2-(4-(2-methoxyphenyl)piperazinyl-1)ethyl)amino)-3(2H)-pyridazinon
Ki = 42.2
2-(2-Dimethylaminoethyl)-5-chlor-4-((2-(4-(2-methylphenyl)piperazinyl-1)ethyl)amino)-3(2H)-pyridazinon
Ki = 15.8
2-Hydroxyethyl-5-chlor-4-((2-(4-(2-methoxyphenyl)piperazinyl-1)ethyl)amino)-3(2H)-pyridazinon
Ki = 2.42
2-Methyl-5-chlor-4-(methyl-N-(3-(4-(2-methoxyphenyl)piperazinyl-1)propyl)amino)-3(2H)-pyridazinon
Ki = 12.9
2-Methyl-5-chlor-4-((3-(4-(2-hydroxy-4-methylphenyl)piperazinyl-1)propyl)amino)-3(2H)-pyridazinon
Ki = 5.34
2-Methyl-5-chlor-4-((3-(4-(2-ethoxy-4-methylphenyl)piperazinyl-1)propyl)amino)-3(2H)-pyridazinon
Ki = 4.99
2-Methyl-5-chlor-4-((3-(4-(2-methylphenyl)piperazinyl-1)propyl)amino)-3(2H)-pyridazinon
Ki = 4.45
2-Methyl-5-chlor-4-((3-(4-(2-fluorophenyl)piperazinyl-1)propyl)amino)-3(2H)-pyridazinon
Ki = 4.89
4-Chlor-5-((2-(4-(2-methoxyphenyl)piperazinyl-1)ethyl)amino)-3(2H)-pyridazinon
Ki = 5.43
2-Methyl-4-chlor-5-((2-(4-phenylpiperazinyl-1)ethyl)amino)-3(2H)-pyridazinon
Ki = 4.05
2-Methyl-4-chlor-5-((2-(4-(2-methoxy-4-methylphenyl)piperazinyl-1)ethyl)amino)-3(2H)-pyridazinon
Ki = 31.4
2-Methyl-4-chlor-5-((2-(4-(2-methoxy-5-methylphenyl)piperazinyl-1)ethyl)amino)-3(2H)-pyridazinon
Ki = 6.98
2-Methyl-4-chlor-5-(ethyl-(2-(4-(2-methoxyphenyl)piperazinyl-1)ethyl)amino)-3(2H)-pyridazinon
Ki = 37.1
2-Methyl-4-chlor-5-((2-(4-(2-ethoxyphenyl)piperazinyl-1)ethyl)amino)-3(2H)-pyridazinon
Ki = 7.59
2-Methyl-4-chlor-5-((2-(4-(2-hydroxyphenyl)piperazinyl-1)ethyl)amino)-3(2H)-pyridazinon
Ki = 15.4
2-Methyl-4-chlor-5-((4-(2-hydroxy-4-methylphenyl)piperazinyl-1)ethyl)amino)-3(2H)-pyridazinon
Ki = 20.8
2-Methyl-4-chlor-5-((2-(4-(2-benzyloxyphenyl)piperazinyl-1)ethyl)amino)-3(2H)-pyridazinon
Ki = 2.0
2-Methyl-4-chlor-5-((2-(4-(2-methylphenyl)piperazinyl-1)ethyl)amino)-3(2H)-pyridazinon
Ki = 6.27
2-Methyl-4-chlor-5-((2-(4-(3-trifluormethylphenyl)piperazinyl-1)ethyl)amino)-3(2H)-pyridazinon
Ki = 94.2
2-Methyl-4-chlor-5-((2-(4-(2-fluorophenyl)piperazinyl-1)ethyl)amino)-3(2H)-pyridazinon
Ki = 28.2
2-t-Butyl-4-chlor-5-((2-(4-(2-methoxyphenyl)piperazinyl-1)ethyl)amino)-3(2H)-pyridazinon
Ki = 12.8
2-(Dimethylaminoethyl)-4-chlor-5-((2-(4-(2-methoxyphenyl)piperazinyl-1)ethyl)amino)-3(2H)-pyridazinon
Ki = 17.3
2-Hydroxyethl-4-chlor-5-((2-(4-(2-methoxyphenyl)piperazinyl-1)ethyl)amino)-3(2H)-pyridazinon
Ki = 10.8
2-Phenyl-4-chlor-5-((2-(4-(2-methoxyphenyl)piperazinyl-1)ethyl)amino)-3(2H)-pyridazinon
Ki = 4.48
2-Methyl-4-chlor-5-(methyl-N-((3-(4-(2-methoxyphenyl)piperazinyl-1)propyl)amino)-3(2H)-pyridazinon
Ki = 3.55
2-Methyl-4-chlor-5-((3-(4-(2-hydroxy-4-methylphenyl)piperazinyl-1)propyl)amino)-3(2H)-pyridazinon
Ki = 4.33
2-Methyl-4-chlor-5-((3-(4-(2-ethoxy-4-methylphenyl)piperazinyl-1)propyl)amino)-3(2H)-pyridazinon
Ki = 3.78
2-Methyl-4-chlor-5-((3-(4-(2-methylphenyl)piperazinyl-1)propyl)amino)-3(2H)-pyridazinon
Ki = 31.4
2-Methyl-4-chlor-5-((3-(4-(2-fluorophenyl)piperazinyl-1)propyl)amino)-3(2H)-pyridazinon
Ki = 32.8
2-Methyl-5-((2-(4-(2-methoxyphenyl)piperazinyl-1)ethyl)amino)-3(2H)pyridazinon
Ki = 190.0
4-((2-(4-(2-Methoxyphenyl)piperazinyl-1)ethyl)amino)-3(2H)-pyridazinon
Ki = 42.2
4-((2-(4-(2-Hydroxyphenyl)piperazinyl-1)ethyl)amino)-3(2H)-pyridazinon
Ki = 84.0.
2-Methyl-4-((2-(4-phenylpiperazinyl-1)ethyl)amino)-3(2H)pyridazinon
Ki = 90.3
2-Methyl-4-((2-(4-(2-methoxyphenyl)piperazinyl-1)ethyl)amino)-3(2H)pyridazinon
Ki = 15.7
2-Methyl-4-(ethyl-(2-(4-(2-methoxyphenyl)piperazinyl-1)ethyl)amino)-3(2H)-pyridazinon
Ki = 68.0
2-Methyl-4-((2-(4-(2-methoxy-5-methylphenyl)piperazinyl-1)ethyl)amino-3(2H)-pyridazinon
Ki = 73.6
2-Methyl-4-((2-(4-(2-ethoxyphenyl)piperazinyl-1)ethyl)amino)-3(2H)pyridazinon
Ki = 13.6
2-Methyl-4-((2-(4-(2-hydroxyphenyl)piperazinyl-1)ethyl)amino)-3(2H)pyridazinon
Ki = 65.5
2-Methyl-4-((2-(4-(2-fluorophenyl)piperazinyl-1)ethyl)amino)-3(2H)-pyridazinon
Ki = 21.4
2-t-Butyl-4-((2-(4-(2-methoxyphenyl)piperazinyl-1)ethyl)amino)-3(2H)pyridazinon
Ki = 60.8
2-Hydroxyethyl-4-((2-(4-(2-methoxyphenyl)piperazinyl-1)ethyl)amino-3(2H)-pyridazinon
Ki = 17.7
4-((3-(4-(2-methoxyphenyl)piperazinyl-1)propyl)amino)-3(2H)-pyridazinon
Ki = 26.1
4-((3-(4-(2-Ethoxyphenyl)piperazinyl-1)propyl)amino)-3(2H)-pyridazinon
Ki = 13.0
2-Methyl-4-((3-(4-(2-methoxyphenyl)piperazinyl-1)propyl)amino-3(2H)-pyridazinon
Ki = 24.7
2-Methyl-4-((3-(4-(2-Hydroxy-4-methylphenyl)piperazinyl-1)propyl)amino)-3(2H)-pyridazinon
Ki = 20.9
2-Methyl-4-((3-(4-(2-ethoxy-4-methylphenyl)piperazinyl-1)propyl)amino)-3(2H)-pyridazinon
Ki = 13.6
2-Methyl-4-((3-(4-(2-fluorophenyl)piperazinyl-1)propyl)amino)-3(2H)-pyridazinon
Ki = 30.2
4-((4-(4-(2-methoxyphenyl)piperazinyl-1)butyl)amino)-3(2H)-pyridazinon
Ki = 4.64
2-Methyl-4-((4-(4-(2-methoxyphenyl)piperazinyl-1)butyl)amino)-3(2H)-pyridazinon
Ki = 11.8
2-Methyl-5-((2-(4-phenylpiperazinyl-1)ethyl)amino)-3(2H)-pyridazinon
Ki = 157.0
2-Methyl-5-((2-(4-(2-methoxy-5-methylphenyl)piperazinyl-1)ethyl)amino)-3(2H)-pyridazinon
Ki = 82.6
2-Methyl-5-(ethyl-(2-(4-(2-methoxyphenyl)piperazinyl-1)ethyl)amino)-3(2H)-pyridazinon
Ki = 20.6
2-Methyl-5-((2-(4-(2-ethoxyphenyl)piperazinyl-1)ethyl)amino)-3(2H)pyridazinon
Ki = 103.0
2-Methyl-5-((2-(4-(2-hydroxyphenyl)piperazinyl-1)ethyl)amino)-3(2H)pyridazinon
Ki = 65.8
2-Methyl-5-((2-(4-(2-methylphenyl)piperazinyl-1)ethyl)amino)-3(2H)-pyridazinon
Ki = 48.4
2-Methyl-4-((2-(4-(2-fluorophenyl)piperazinyl-1)ethyl)amino)-3(2H)-pyridazinon
Ki = 89.4
2-t-Butyl-5-((2-(4-(2-methoxyphenyl)piperazinyl-1)ethyl)amino)-3(2H)pyridazinon
Ki = 6.03
2-Hydroxyethyl-5-((2-(4-(2-methoxyphenyl)piperazinyl -1)ethyl)amino)-3(2H)-pyridazinon
Ki = 128.0
2-Phenyl-5-((2-(4-(2-methoxyphenyl)piperazinyl-1)ethyl)amino)-3(2H)-pyridazinon
Ki = 61.6
2-Methyl-4-((3-(4-(2-methoxyphenyl)piperazinyl-1)propyl)amino-3(2H)-pyridazinon
Ki = 40.5
2-Methyl-5-((3-(4-(2-ethoxy-4-methylphenyl)piperazinyl-1)propyl)amino)-3(2H)-pyridazinon
Ki = 23.3
2-Methyl-4-((3-(4-(2-methylphenyl)piperazinyl-1)propyl)amino)-3(2H)-pyridazinon
Ki = 49.3
2-Methyl-5-((4-(4-(2-methoxyphenyl)piperazinyl-1)butyl)amino)-3(2H)-pyridazinon
Ki = 5.38
2-Methyl-5-methoxy-4-((2-(4-(2-methoxyphenyl)piperazinyl-1)ethyl)amino)-3(2H)-pyridazinon
Ki = 7.83
4-Chlor-5-((2-(4-(2-methoxyphenyl)piperazinyl-1)ethyl)amino)-3(2H)-pyridazinon
Ki = 5.43
6-Chlor-2-methyl-4-((3-(4-(2-methoxyphenyl)piperazinyl-1)propyl)amino)-3-(2H)-pyridazinon
Ki = 11.0
6-Chlor-2-ethyl-4-((3-(4-(2-methoxyphenyl)piperazinyl-1)propyl)amino)-3(2H)-pyridazinon
Ki = 18.0
6-Chlor-4-((4-(4-(2-methoxyphenyl)piperazinyl-1)butyl)amino)-3(2H)-pyridazinon
Ki = 3.34
6-Chlor-4-((2-(4-(2-methoxyphenyl)piperazinyl-1)ethyl)amino)-3(2H)-pyridazinon
Ki = 12.8
2-Methyl-6-chlor-4-((2-(4-(2-methoxyphenyl)piperazinyl-1)ethyl)amino)-3(2H)-pyridazinon
Ki = 5.63
6-Chlor-4-((2-(4-(2-iso-propoxyphenyl)piperazinyl-1)ethyl)amino)-3(2H)-pyridazinon
Ki = 10.0
6-Chlor-4-((3-(4-(2-methoxyphenyl)piperazinyl-1)propyl)amino)-3(2H)-pyridazinon
Ki = 11.0
6-Chlor-4-((3-(4-(2-methoxy-4-methylphenyl)piperazinyl-1)propyl)amino)-3(2H)pyridazinon
Ki = 7.49
2-Methyl-6-chlor-3-((3-(4-(2-ethoxyphenyl)piperazinyl-1)propyl)amino)-3(2H)-pyridazinon
Ki = 6.5
6-Chlor-2-hydroxyethyl-4-((3-(4-(2-methoxyphenyl)piperazinyl-1)propyl)amino)-3(2H)pyridazinon
Ki = 9.6
Vergleichssubstanz:
6-(3-(4-(2-Methoxyphenyl)piperazinyl-1)propyl)amino)-1,3-di-methyluracil (URAPIDIL)
Ki = 110.0

### Beispiel B:

### Bestimmung der Affinität von Verbindungen der Formel I zu 5-HT-1A Rezeptoren.

Die Affinität von Verbindungen der allgemeinen Formel I zu 5-HT-1A Rezeptoren wurde nach der von H. Gozlan, S. Elmestikawy, L. Pichat, J. Glowinski und M. Hamon in Nature 305, 140 - 142 (1983) beschriebenen Methode bestimmt. Nach dieser methode mißt man die kompetitive Verdrängung von tritiiertem 8-OH-DPAT (8-Hydroxy-(di-n-propylamino)tetralin) an Rattenhirnmembranen durch die Testsubstanzen und ermittelt als IC₅₀ (50 % inhibition concentration) diejenige Konzentration, welche eine 50 %ige Hemmung der spezifischen Bindung des tritiierten 8-OH-DPAT an 5-HT-1A Rezeptoren in Rattenhirnmembranen bewirkt.

Aus den IC₅₀-Werten wurden die konzentrationsunabhängigen Inhibitorkonstanten K_{I}-Alpha₁ und K_{I}-5HT-1A entsprechend den Angaben von Y. Cheng und H.W. Prusoff in Biochem.Pharmacol. 22, 3099 - 3108 (1973) ermittelt.

Die Ergebnisse dieser Untersuchungen sind in der folgenden Tabelle zusammengefaßt:

### Tabelle II

### Inhibitionskonstanten am 5-HT1A-Rezeptor:

2-Methyl-5-brom-4-((2-(4-(2-methoxyphenyl)piperazin-1)ethyl)amino)-3(2H)-pyridazinon
Ki = 16.2
2-Methyl-4-brom-5-((2-(4-(2-methoxyphenyl)piperazinyl-1)ethyl)amino)-3(2H)-pyridazinon
Ki = 33.6
2-Methyl-5-chlor-4-((2-(4-(2-methoxyphenyl)piperazinyl-1)ethyl)amino)-3(2H)-pyridazinon
Ki = 16.6
2-Methyl-4-chlor-5-((2-(4-(2-methoxyphenyl)piperazinyl-1)ethyl)amino)-3(2H)-pyridazinon
Ki = 63.2
2-Methyl-4-chlor-5-((3-(4-(2-methoxyphenyl)piperazinyl-1)propyl)amino)-3(2H)-pyridazinon
Ki = 50.7
2-Methyl-5-chlor-4-((3-(4-(2-methoxyphenyl)piperazinyl-1)propyl)amino)-3(2H)-pyridazinon
Ki = 1.40
2-Methyl-5-chlor-4-((2-(4-(2-methoxy-5-methylphenyl)piperazinyl-1)ethyl)amino)-3(2H)-pyridazinon
Ki = 85.1
2-Methyl-5-chlor-4-((2-(4-(2-methoxy-4-methylphenyl)piperazinyl-1)ethyl)amino)-3(2H)-pyridazinon
Ki = 126.0
2-Methyl-5-chlor-4-((2-(4-(3-trifluormethylphenyl)piperazinyl-1)ethyl)amino-3(2H)-pyridazinon
Ki = 8.31
2-Methyl-5-chlor-4-((2-(4-(2-fluorophenyl)piperazinyl-1)ethyl)amino)-3(2H)-pyridazinon
Ki = 80.8
2-t-Butyl-5-chlor-4-((2-(4-(2-methoxyphenyl)piperazinyl-1)ethyl)amino)-3(2H)pyridazinon
Ki = 9.45
2-Hydroxyethyl-5-chlor-4-((2-(4-(2-methoxyphenyl)piperazinyl-1)ethyl)amino)-3(2H)-pyridazinon
Ki = 25.6
4-Chlor-5-((2-(4-(2-methoxyphenyl)piperazinyl-1)ethyl)amino)-3(2H)-pyridazinon
Ki = 27.0
2-Methyl-4-chlor-5-((2-(4-phenylpiperazinyl-1)ethyl)amino)-3(2H)-pyridazinon
Ki = 106.0
2-Methyl-4-chlor-5-((2-(4-(2-phenyl)piperazinyl-1)ethyl)amino)-3(2H)-pyridazinon
Ki = 36.8
2-Methyl-4-chlor-5-((2-(4-(3-trifluormethylphenyl)piperazinyl-1)ethyl)amino-3(2H)-pyridazinon
Ki = 28.5
2-t-Butyl-4-chlor-5-((2-(4-(2-methoxyphenyl)piperazinyl-1)ethyl)amino)-3(2H)-pyridazinon
Ki = 39.4
2-(Dimethylaminoethyl)-4-chlor-5-((2-(4-(2-methoxyphenyl)piperazinyl-1)ethyl)amino)-3(2H)-pyridazinon
Ki = 118
2-Hydroxyethl-4-chlor-5-((2-(4-(2-methoxyphenyl)piperazinyl-1-)ethyl)amino-3(2H)-pyridazinon
Ki = 86.3
2-Phenyl-4-chlor-5-((2-(4-(2-methoxyphenyl)piperazinyl-1)ethyl)amino)-3(2H)-pyridazinon
Ki = 75.8
2-Methyl-5-((2-(4-(2-methoxyphenyl)piperazinyl-1)ethyl)amino)-3(2H)pyridazinon
Ki = 43.6
2-Methyl-4-(ethyl-(2-(4-(2-methoxyphenyl)piperazinyl-1)ethyl)amino)-3(2H)-pyridazinon
Ki = 40.7
2-Methyl-4-((2-(4-(2-methoxy-5-methylphenyl)piperazinyl-1)ethyl)amino)-3(2H)-pyridazinon
Ki = 186.0
2-Methyl-4-((2-(4-(2-hydroxyphenyl)piperazinyl-1)ethyl)amino)-3(2H)pyridazinon
Ki = 36.5
2-Methyl-4-((2-(4-(3-trifluormethylphenyl)piperazinyl-1)ethyl)amino-3(2H)-pyridazinon
Ki = 4.56
2-Hydroxyethyl-4-((2-(4-(2-methoxyphenyl)piperazinyl-1)ethyl)amino-3(2H)-pyridazinon
Ki = 18.2
2-Methyl-4-((3-(4-(2-methoxyphenyl)piperazinyl-1)propyl)amino-3(2H)-pyridazinon
Ki = 15.9
2-Methyl-5-(ethyl-(2-(4-(2-methoxyphenyl)piperazinyl-1)ethyl)amino)-3(2H)-pyridazinon
Ki = 24.2
2-t-Butyl-5-((2-(4-(2-methoxyphenyl)piperazinyl-1)ethyl)amino)-3(2H)pyridazinon
Ki = 65.8
2-Hydroxyethyl-5-((2-(4-(2-methoxyphenyl)piperazinyl -1)ethyl)amino)-3(2H)-pyridazinon
Ki = 55.8
2-Methyl-4-((3-(4-(2-methoxyphenyl)piperazinyl-1)propyl)amino-3(2H)-pyridazinon
Ki = 55.3
2-Methyl-5-methoxy-4-((2-(4-(2-methoxyphenyl)piperazinyl-1)ethyl)amino)-3(2H)-pyridazinon
Ki = 90.8
4-Chlor-5-((2-(4-(2-methoxyphenyl)piperazinyl-1)ethyl)amino)-3(2H)-pyridazinon
Ki = 27.0
6-Chlor-2-methyl-4-((3-(4-(2-methoxyphenyl)piperazinyl-1)propyl)amino)-3(2H)-pyridazinon
Ki = 46.6
2-Methyl-6-chlor-4-((2-(4-(2-methoxyphenyl)piperazinyl-1)ethyl)amino)-3(2H)-pyridazinon
Ki =28.4
Vergleichssubstanz:

6-(3-(4-(2-Methoxyphenyl)piperazinyl-1)propyl)amino)-1,3-dimethyluracil (URAPIDIL)
Ki= 93.1

## Patentansprüche (Patentansprüche für folgende(n) Vertragsstaat(en): AT, BE, CH, DE, FR, GB, IT, LI, LU, NL, SE)

1. Piperazinylalkyl-3(2H)-pyridazinone der Formel 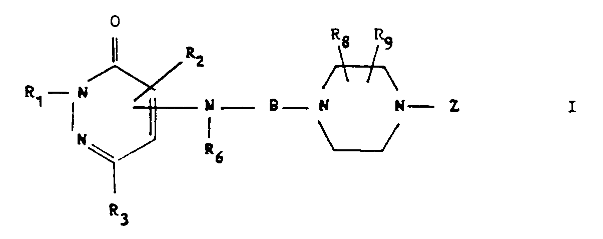 in der die Reste R₁ Wasserstoff, Phenyl, Benzyl, unsubstituiertes oder ein- oder mehrfach durch Hydroxy, Piperidin, Morpholin oder durch eine Gruppe NR₄R₅, in der R₄ und R₅ gleich oder verschieden sein können und Wasserstoff, Methyl oder Ethyl darstellen, substituiertes (C₁ - C₆)-Alkyl,
R₂ und R₃ Wasserstoff, Halogen, (C₁ - C₆)-Alkoxy oder (C₁ - C₆)-Alkyl, wobei mindestens einer der Reste R₂ oder R₃ Wasserstoff bedeutet,
R₆ Wasserstoff, (C₁ - C₄)-Alkyl, Phenyl, Benzyl oder Phenylethyl,
B unsubstituiertes oder ein- oder mehrfach durch Hydroxy, (C₁ - C₄)-Alkyl oder durch die Gruppe NR₄R₅ substituiertes (C₁ - C₇)-Alkylen, welches gegebenenfalls zu einem alicyclischen 4 bis 7-Ring geschlossen sein kann,
R₈ und R₉, die gleich oder verschieden sein können, Wasserstoff oder (C₁ - C₆)-Alkyl und
Z unsubstituiertes oder ein- oder mehrfach durch (C₁ - C₆)-Alkyl, (C₁ -C₆)-Alkoxy, Benzyloxy, Trifluormethyl, Halogen, Nitro, (C₃ - C₇)-Cycloalkoxy, (C₁ - C₄)-Alkylthio, Trifluormethylthio oder durch die Gruppe NR₄R₅ substituiertes Phenyl, Naphthyl, Pyridyl oder Thiazolyl darstellen, und ihre pharmazeutisch verwendbaren Salze.

2. Verbindungen der Formel I nach Anspruch 1, worin die Reste
R₁ Wasserstoff, Methyl, Ethyl, tert.-Butyl, Benzyl, 2-Hydroxyethyl oder 2-Dimethylaminoethyl,
R₂ und R₃ Wasserstoff, Chlor, Brom oder Methoxy, wobei mindestens einer der Reste R₂ oder R₃ Wasserstoff bedeutet,
R₆ Wasserstoff, Methyl oder Ethyl,
B geradkettiges (C₂ - C₆)-Alkylen,
R₈ und R₉ Wasserstoff und
Z unsubstituiertes oder ein- oder mehrfach durch Methyl, (C₁ - C₃)-Alkoxy, Benzyloxy, Trifluormethyl, Fluor, Chlor oder Nitro substituiertes Phenyl oder unsubstituiertes Pyridyl-2 darstellen, sowie ihre pharmazeutisch verwendbaren Salze.

3. 2-Methyl-4-chlor-5-((2-(4-(2-methoxyphenyl)piperazinyl-1)ethyl)amino)-3(2H)pyridazinon.

4. Verfahren zur Herstellung von Verbindungen der in Anspruch 1 definierten Formel I und von deren pharmazeutisch verwendbaren Salzen, dadurch gekennzeichnet, daß man
a) eine Verbindung der Formel 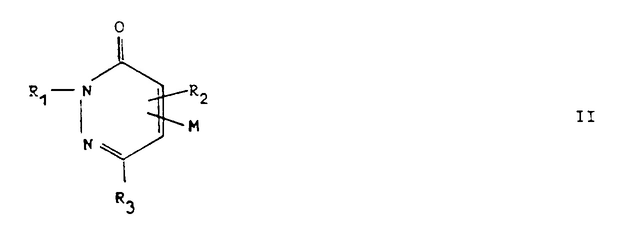 in der R₁, R₂ und R₃ wie oben definiert sind und M eine Abgangsgruppe bedeutet, mit einer Verbindung der Formel 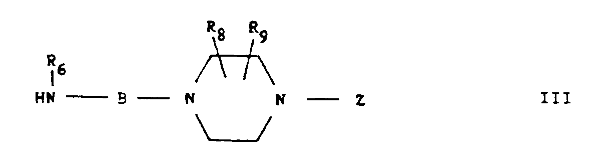 in der R₆, B, R₈, R₉ und Z wie oben definiert sind, umsetzt, oder
b) in einer Verbindung der Formel I, in der einer der Reste R₂ oder R₃ Halogen darstellt und die übrigen Reste wie oben definiert sind, das Halogen von R₂ oder R₃ mittels hydrierender Enthalogenierung durch Wasserstoff ersetzt, oder
c) eine Verbindung der Formel I, in der einer der Reste R₂ oder R₃ Halogen darstellt und die übrigen Reste wie oben definiert sind, mit einem Alkalimetallalkoholat umsetzt, wobei das Halogen von R₂ oder R₃ in einen Rest mit der Bedeutung (C₁ - C₆)-Alkoxy umgewandelt wird, oder
d) in einer Verbindung der Formel I, in der R₁ die Bedeutung von i-Propyl, sec. Butyl, t-Butyl oder Benzyl hat und die übrigen Reste wie oben definiert sind, R₁ durch Säuren abspaltet oder
e) ein Pyridazin der Formel 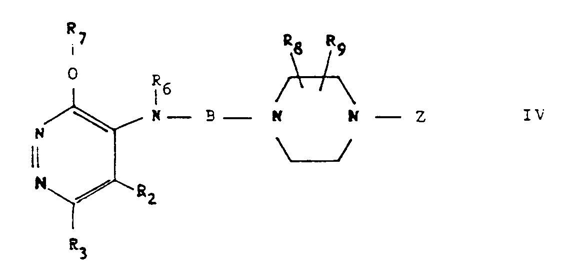 in der R₂, R₃, R₆, B, R₈, R₉ und Z wie oben definiert sind und R₇ die Bedeutung von (C₁ - C₆)-Alkyl hat, unter Etherspaltung mit Säure in das entsprechende 3(2H)-Pyridazinon überführt oder
f) eine Verbindung der Formel I, in der R₁ Wasserstoff und einer der Reste R₂ oder R₃ Halogen darstellt und die übrigen Reste wie oben definiert sind, durch Reaktion mit einem alkylierenden Reagens in Position 2 des Pyridazin-Ringes alkyliert und
g) gewünschtenfalls eine nach a) bis e) erhaltene Verbindung der Formel I in ihre pharmazeutisch verträglichen Salze überführt.

5. Pharmazeutische Präparate, enthaltend Verbindungen der Formel I oder ein Salz davon nach Anspruch 1 in Kombination mit üblichen galenischen Hilfs-und/oder Trägerstoffen oder Verdünnungsmitteln.

6. Pharmazeutische Präparate, enthaltend Verbindungen der Formel I oder ein Salz davon nach Anspruch 1 in Kombination mit anderen therapeutisch wertvollen Wirkstoffen sowie üblichen galenischen Hilfs- und/oder Trägerstoffen oder Verdünnungsmitteln.

7. Verbindungen der Formel I oder Salze davon nach Anspruch 1 zur Verwendung als Wirkstoffe für Arzneimittel zur Behandlung von Hypertonie, Herzinsuffizienz und/oder peripheren Kreislaufstörungen.

## Patentansprüche (Patentansprüche für folgende(n) Vertragsstaat(en): ES, GR)

1. Verfahren zur Herstellung neuer Piperazinylalkyl-3(2H)-pyridazinone der Formel 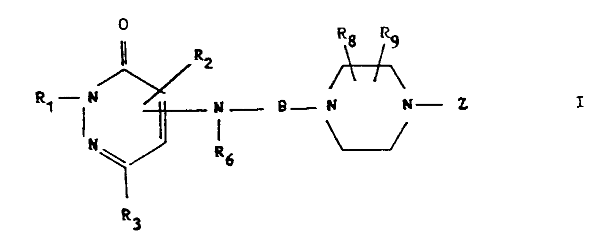 in der die Reste R₁ Wasserstoff, Phenyl, Benzyl, unsubstituiertes oder ein- oder mehrfach durch Hydroxy, Piperidin, Morpholin oder durch eine Gruppe NR₄R₅, in der R₄ und R₅ gleich oder verschieden sein können und Wasserstoff, Methyl oder Ethyl darstellen, substituiertes (C₁ - C₆)-Alkyl,
R₂ und R₃ Wasserstoff, Halogen, (C₁ - C₆)-Alkoxy oder (C₁ - C₆)-Alkyl, wobei mindestens einer der Reste R₂ oder R₃ Wasserstoff bedeutet,
R₆ Wasserstoff, (C₁ - C₄)-Alkyl, Phenyl, Benzyl oder Phenylethyl,
B unsubstituiertes oder ein- oder mehrfach durch Hydroxy, (C₁ - C₄)-Alkyl oder durch die Gruppe NR₄R₅ substituiertes (C₁ - C₇)-Alkylen, welches gegebenenfalls zu einem alicyclischen 4 bis 7-Ring geschlossen sein kann,
R₈ und R₉, die gleich oder verschieden sein können, Wasserstoff oder (C₁ - C₆)-Alkyl und
Z unsubstituiertes oder ein- oder mehrfach durch (C₁ - C₆)-Alkyl, (C₁ -C₆)-Alkoxy, Benzyloxy, Trifluormethyl, Halogen, Nitro, (C₃ - C₇)-Cycloalkoxy, (C₁ - C₄)-Alkylthio, Trifluormethylthio oder durch die Gruppe NR₄R₅ substituiertes Phenyl, Naphthyl, Pyridyl oder Thiazolyl darstellen, und von ihren pharmazeutisch verwendbaren Salzen, dadurch gekennzeichnet, daß man
a) eine Verbindung der Formel 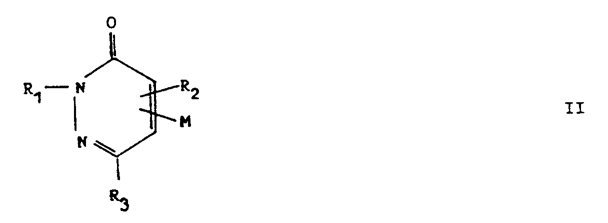 in der R₁, R₂ und R₃ wie oben definiert sind und M eine Abgangsgruppe bedeutet, mit einer Verbindung der Formel 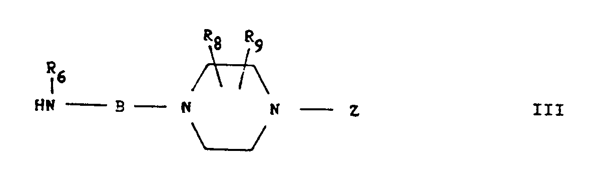 in der R₆, B, R₈, R₉ und Z wie oben definiert sind, umsetzt, oder
b) in einer Verbindung der Formel I, in der einer der Reste R₂ oder R₃ Halogen darstellt und die übrigen Reste wie oben definiert sind, das Halogen von R₂ oder R₃ mittels hydrierender Enthalogenierung durch Wasserstoff ersetzt, oder
c) eine Verbindung der Formel I, in der einer der Reste R₂ oder R₃ Halogen darstellt und die übrigen Reste wie oben definiert sind, mit einem Alkalimetallalkoholat umsetzt, wobei das Halogen von R₂ oder R₃ in einen Rest mit der Bedeutung (C₁ - C₆)-Alkoxy umgewandelt wird, oder
d) in einer Verbindung der Formel I, in der R₁ die Bedeutung von i-Propyl, sec. Butyl, t-Butyl oder Benzyl hat und die übrigen Reste wie oben definiert sind, R₁ durch Säuren abspaltet oder
e) ein Pyridazin der Formel 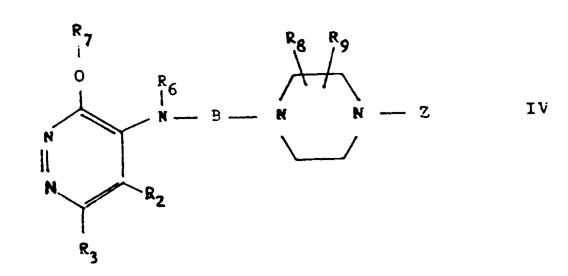 in der R₂, R₃, R₆, B, R₈, R₉ und Z wie oben definiert sind und R₇ die Bedeutung von (C₁ - C₆)-Alkyl hat, unter Etherspaltung mit Säure in das entsprechende 3(2H)-Pyridazinon überführt oder
f) eine Verbindungen der Formel I, in der R₁ Wasserstoff und einer der Reste R₂ oder R₃ Halogen darstellt und die übrigen Reste wie oben definiert sind, durch Reaktion mit einem alkylierenden Reagens in Position 2 des Pyridazin-Ringes alkyliert und
g) gewünschtenfalls eine nach a) bis e) erhaltene Verbindung der Formel I in ihre pharmazeutisch verträglichen Salze überführt.

2. Verfahren zur Herstellung pharmazeutischer Präparate, dadurch gekennzeichnet, daß man eine Verbindung der Formel I oder ein Salz davon nach Anspruch 1 mit üblichen galenischen Hilfs- und /oder Trägerstoffen oder Verdünngungsmitteln kombiniert.

3. Verwendung von Verbindungen der Formel I oder ihrer Salze nach Anspruch 1 zur Herstellung von Arzneimitteln zur Behandlung von Hypertonie, Herzinsuffizienz und/oder peripheren Kreislaufstörungen.

## Claims (Claims for the following Contracting State(s): AT, BE, CH, DE, FR, GB, IT, LI, LU, NL, SE)

1. Piperazinylalkyl-3(2H)-pyridazinones of the formula 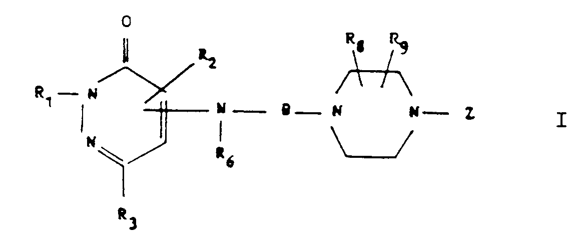 in which the radicals
R₁ represents hydrogen, phenyl, benzyl, or (C₁-C₆)-alkyl which is unsubstituted or substituted one or more times by hydroxyl, piperidine, morpholine or by a group NR₄R₅ in which R₄ and R₅ can be identical or different and represent hydrogen, methyl or ethyl,
R₂ and R₃ represent hydrogen, halogen, (C₁-C₆)-alkoxy or (C₁-C₆)-alkyl where at least one of the radicals R₂ or R₃ denotes hydrogen,
R₆ represents hydrogen, (C₁-C₄)-alkyl, phenyl, benzyl or phenylethyl,
B represents (C₁-C₇)-alkylene which is unsubstituted or substituted one or more times by hydroxyl, (C₁-C₄)-alkyl or by the group NR₄R₅ and which can optionally be closed to an alicyclic 4 to 7-membered ring,
R₈ and R₉, which can be identical or different, represent hydrogen or (C₁-C₆)-alkyl and
Z represents phenyl, naphthyl, pyridyl or thiazolyl, each of which is unsubstituted or substituted one or more times by (C₁-C₆)-alkyl, (C₁-C₆)-alkoxy, benzyloxy, trifluoromethyl, halogen, nitro, (C₃-C₇)-cycloalkoxy, (C₁-C₄)-alkylthio, trifluoromethylthio or by the group NR₄R₅, and the pharmaceutically utilisable salts thereof.

2. Compounds of the formula I according to Claim 1, in which the radicals
R₁ represents hydrogen, methyl, ethyl, tert.-butyl, benzyl, 2-hydroxyethyl or 2-dimethylaminoethyl,
R₂ and R₃ represent hydrogen, chlorine, bromine or methoxy, where at least one of the radicals R₂ or R₃ denotes hydrogen,
R₆ represents hydrogen, methyl or ethyl,
B represents straight-chain (C₂-C₆)-alkylene,
R₈ and R₉ represent hydrogen and
Z represents phenyl which is unsubstituted or substituted one or more times by methyl, (C₁-C₃)-alkoxy, benzyloxy, trifluoromethyl, fluorine, chlorine or nitro, or unsubstituted 2-pyridyl, and the pharmaceutically utiisable salts thereof.

3. 2-Methyl-4-chloro-5-((2-(4-(2-methoxyphenyl)-1-piperazinyl)ethyl)amino)-3(2H)-pyridazinone.

4. Process for the preparation of compounds of the formula I defined in Claim 1 and of the pharmaceutically utilisable salts thereof, characterised in that
a) a compound of the formula 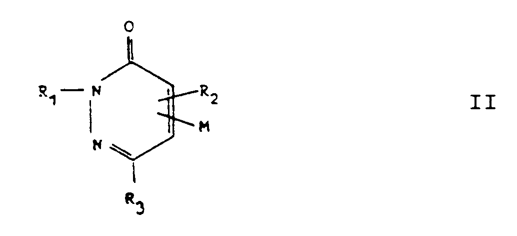 in which R₁, R₂ and R₃ are as defined above, and M denotes a leaving group, is reacted with a compound of the formula 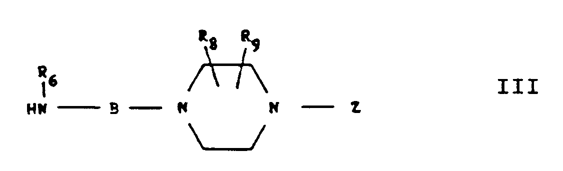 in which R₆, B, R₈, R₉ and Z are as defined above, or
b) in a compound of the formula I in which one of the radicals R₂ or R₃ represents halogen, and the other radicals are as defined above, the halogen of R₂ or R₃ is replaced by hydrogen by means of hydrogenating dehalogenation, or
c) a compound of the formula I in which one of the radicals R₂ or R₃ represents halogen, and the other radicals are as defined above, is reacted with an alkali metal alcoholate, with the halogen of R₂ or R₃ being converted into a radical with the meaning (C₁-C₆)-alkoxy, or
d) in a compound of the formula I in which R₁ has the meaning of i-propyl, sec.butyl, t-butyl or benzyl, and the other radicals are as defined above, R₁ is eliminated by acids or
e) a pyridazine of the formula 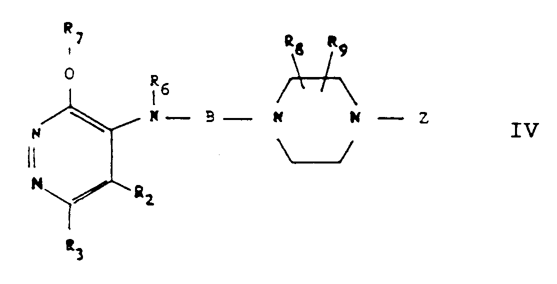 in which R₂, R₃, R₆, B, R₈, R₉ and Z are as defined above, and R₇ has the meaning of (C₁-C₆)-alkyl, is converted by ether cleavage with acid into the corresponding 3(2H)-pyridazinone or
f) a compound of the formula I in which R₁ represents hydrogen and one of the radicals R₂ or R₃ represents halogen, and the other radicals are as defined above, is alkylated in position 2 of the pyridazine ring by reaction with an alkylating reagent and
g) if required a compound of the formula I obtained as in a) to e) is converted into the pharmaceutically compatible salts thereof.

5. Pharmaceutical products containing compounds of the formula I or a salt thereof according to Claim 1 in combination with conventional pharmaceutical ancillary substances and/or excipients or diluents.

6. Pharmaceutical products containing compounds of the formula I or a salt thereof according to Claim 1 in combination with other active substances of therapeutic value, as well as conventional pharmaceutical ancillary substances and/or excipients or diluents.

7. Compounds of the formula I or salts thereof according to Claim 1 for use as active substances for pharmaceuticals for the treatment of hypertension, heart failure and/or peripheral circulatory disorders.

## Claims (Claims for the following Contracting State(s): ES, GR)

1. Process for the preparation of novel piperazinylalkyl-3(2H)-pyridazinones of the formula 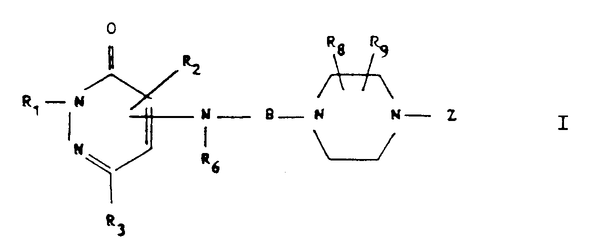 in which the radicals
R₁ represents hydrogen, phenyl, benzyl, or (C₁-C₆)-alkyl which is unsubstituted or substituted one or more times by hydroxyl, piperidine, morpholine or by a group NR₄R₅ in which R₄ and R₅ can be identical or different and represent hydrogen, methyl or ethyl,
R₂ and R₃ represent hydrogen, halogen, (C₁-C₆)-alkoxy or (C₁-C₆)-alkyl where at least one of the radicals R₂ or R₃ denotes hydrogen,
R₆ represents hydrogen, (C₁-C₄)-alkyl, phenyl, benzyl or phenylethyl,
B represents (C₁-C₇)-alkylene which is unsubstituted or substituted one or more times by hydroxyl, (C₁-C₄)-alkyl or by the group NR₄R₅ and which can optionally be closed to an alicyclic 4 to 7-membered ring,
R₈ and R₉, which can be identical or different, represent hydrogen or (C₁-C₆)-alkyl and
Z represents phenyl, naphthyl, pyridyl or thiazolyl, each of which is unsubstituted or substituted one or more times by (C₁-C₆)-alkyl, (C₁-C₆)-alkoxy, benzyloxy, trifluoromethyl, halogen, nitro, (C₃-C₇)-cycloalkoxy, (C₁-C₄)-alkylthio, trifluoromethylthio or by the group NR₄R₅, and the pharmaceutically utilisable salts thereof, characterised in that
a) a compound of the formula 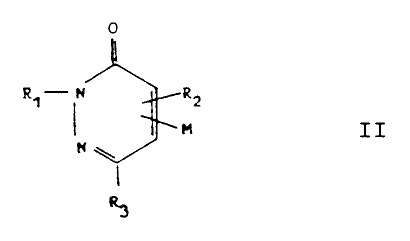 in which R₁, R₂ and R₃ are as defined above, and M denotes a leaving group, is reacted with a compound of the formula 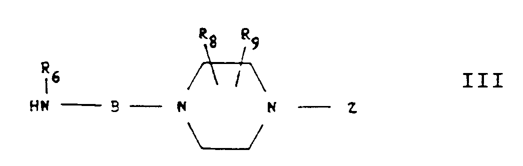 in which R₆, B, R₈, R₉ and Z are as defined above, or
b) in a compound of the formula I in which one of the radicals R₂ or R₃ represents halogen, and the other radicals are as defined above, the halogen of R₂ or R₃ is replaced by hydrogen by means of hydrogenating dehalogenation, or
c) a compound of the formula I in which one of the radicals R₂ or R₃ represents halogen, and the other radicals are as defined above, is reacted with an alkali metal alcoholate, with the halogen of R₂ or R₃ being converted into a radical with the meaning (C₁-C₆)-alkoxy, or
d) in a compound of the formula I in which R₁ has the meaning of i-propyl, sec.butyl, t-butyl or benzyl, and the other radicals are as defined above, R₁ is eliminated by acids or
e) a pyridazine of the formula 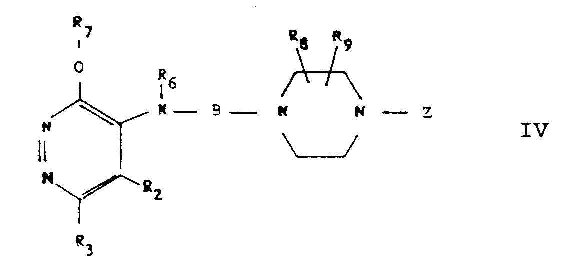 in which R₂, R₃, R₆, B, R₈, R₉ and Z are as defined above, and R₇ has the meaning of (C₁-C₆)-alkyl, is converted by ether cleavage with acid into the corresponding 3(2H)-pyridazinone or
f) a compound of the formula I in which R₁ represents hydrogen and one of the radicals R₂ or R₃ represents halogen, and the other radicals are as defined above, is alkylated in position 2 of the pyridazine ring by reaction with an alkylating reagent and
g) if required a compound of the formula I obtained as in a) to e) is converted into the pharmaceutically compatible salts thereof.

2. Process for the preparation of pharmaceutical products, characterised in that a compound of the formula I or a salt thereof according to Claim 1 is combined with conventional pharmaceutical ancillary substances and/or excipients or diluents.

3. Use of compounds of the formula I or the salts thereof, according to Claim 1 for the preparation of pharmaceuticals for the treatment of hypertension, heart failure and/or peripheral circulatory disorders.

## Revendications (Revendications pour l'(les) Etat(s) contractant(s) suivant(s): AT, BE, CH, DE, FR, GB, IT, LI, LU, NL, SE)

1. Pipérazinylalcoyl-3(2H)-pyridazinones de formule 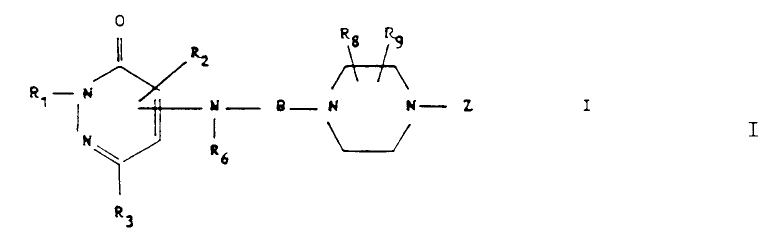 dans laquelle
R₁ représente l'atome d'hydrogène, un reste phényle, benzyle ou alcoyle en C₁-C₆ non substitué ou substitué une ou plusieurs fois par un groupe hydroxy, pipéridine, morpholine ou par un groupe NR₄R₅ dans lequel R₄ et R₅ peuvent être identiques ou différents et représentent l'atome d'hydrogène ou un groupe méthyle ou éthyle ;
R₂ et R₃ représentent l'atome d'hydrogène, un atome d'halogène, un groupe alcoxy en C₁₋C₆ ou un groupe alcoyle en C₁-C₆, au moins un des restes R₂ ou R₃ signifiant l'atome d'hydrogène ;
R₆ représente l'atome d'hydrogène, un groupe alcoyle en C₁-C₄, phényle, benzyle ou phényléthyle ;
B représente un groupe alcoylène en C₁-C₇ non substitué ou substitué une ou plusieurs fois par un groupe hydroxy, alcoyle en C₁-C₄ ou par le groupe NR₄R₅, qui peut éventuellement être fermé en un composé alicyclique à 4 à 7 chaînons ;
R₈ et R₉, qui peuvent être identiques ou différents, représentent l'atome d'hydrogène ou un groupe alcoyle en C₁-C₆ ; et
Z représente un groupe phényle, naphtyle, pyridyle ou thiazolyle, non substitué ou substitué une ou plusieurs fois par un groupe alcoyle en C₁-C₆, alcoxy en C₁-C₆, benzyloxy, trifluorométhyle, halogéno, nitro, cycloalcoxy en C₃-C₇, alcoylthio en C₁-C₄, trifluorométhylthio ou par le groupe NR₄R₅,
et leurs sels pharmaceutiquement utilisables.

2. Composés de formule I selon la revendication 1, dans lesquels
R₁ représente l'atome d'hydrogène, un groupe méthyle, éthyle, tert-butyle, benzyle, 2-hydroxyéthyle ou 2-diméthylaminoéthyle,
R₂ et R₃ représentent l'atome d'hydrogène, de chlore ou de brome ou un groupe méthoxy, au moins un des restes R₂ ou R₃ signifiant l'atome d'hydrogène,
R₆ représente l'atome d'hydrogène ou un groupe méthyle ou éthyle,
B représente un groupe alcoylène linéaire en C₂-C₆,
R₈ et R₉ représentent chacun l'atome d'hydrogène, et
Z représente un groupe phényle non substitué ou substitué une ou plusieurs fois par un groupe méthyle, alcoxy en C₁-C₃, benzyloxy, trifluorométhyle, fluoro, chloro ou nitro, ou le groupe 2-pyridyle non substitué,
ainsi que leurs sels pharmaceutiquement utilisables.

3. 2-méthyl-4-chloro-5-((2-(4-(2-méthoxyphényl)pipérazinyl-1)éthyl)amino)-3(2H)-pyridazinone.

4. Procédé de préparation des composés répondant à la formule I définie dans la revendication 1, et de leurs sels pharmaceutiquement utilisables, caractérisé en ce que
a) on fait réagir un composé de formule 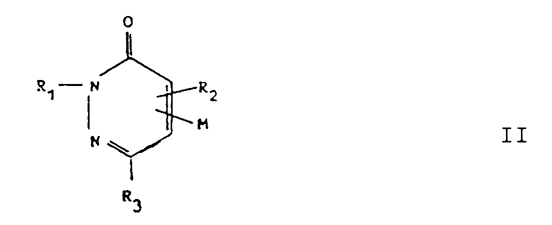 dans laquelle R₁, R₂ et R₃ sont définis comme indiqué ci-dessus et M signifie un groupe de départ, avec un composé de formule 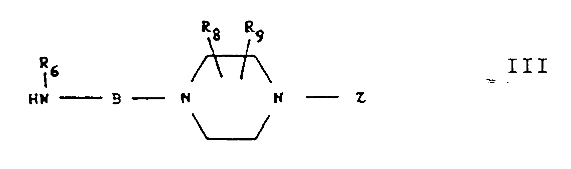 dans lequel R₆, B, R₈, R₉ et Z ont la signification indiquée ci-dessus, ou
b) on remplace, dans un composé de formule I, dans laquelle un des restes R₂ ou R₃ représente un atome d'halogène et les autres restes sont définis comme indiqué ci-dessus, l'atome d'halogène de R₂ ou R₃ par un atome d'hydrogène, par hydrodéshalogénation, ou
c) on fait réagir un composé de formule I, dans laquelle un des restes R₂ ou R₃ représente un atome d'halogène et les autres restes sont définis comme indiqué ci-dessus, avec un alcoolate de métal alcalin, l'atome d'halogène de R₂ ou R₃ étant transformé en un reste ayant la signification d'un groupe alcoxy en C₁-C₆, ou
d) on sépare R₁ par des acides dans un composé de formule I dans laquelle R₁ signifie un groupe i-propyle, sec.-butyle, t-butyle ou benzyle, et les autres restes sont définis comme indiqué ci-dessus, ou
e) on transforme une pyridazine de formule 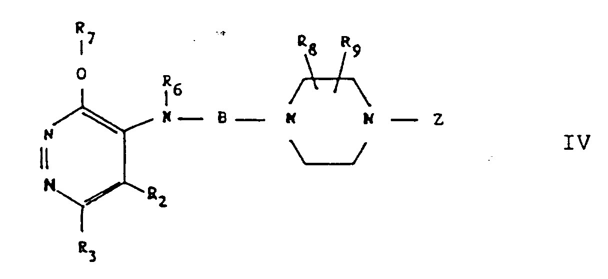 dans laquelle R₂, R₃, R₆, R₈, R₉ et Z sont définis comme indiqué ci-dessus et R₇ signifie un groupe alcoyle en C₁-C₆, avec séparation d'éther avec un acide, en la 3(2H)-pyridazinone correspondante, ou
f) on alcoyle un composé de formule I, dans laquelle R₁ représente l'atome d'hydrogène, un des restes R₂ ou R₃ représente un atome d'halogène et les autres restes sont définis comme indiqué ci-dessus, par réaction avec un réactif alcoylant en position 2 du cycle pyridazinique, et
g) si on le souhaite, on transforme un composé de formule I obtenu d'après a) à e) en ses sels pharmaceutiquement acceptables.

5. Préparations pharmaceutiques contenant des composés de formule I, ou un de leurs sels, selon la revendication 1, combinés avec des adjuvants et/ou des excipients ou des diluants galéniques usuels.

6. Préparations pharmaceutiques contenant des composés de formule I ou un de leurs sels, selon la revendication 1, combinés avec d'autres principes actifs précieux du point de vue thérapeutique, ainsi qu'avec des adjuvants et/ou des excipients ou des diluants galéniques usuels.

7. Composés de formule I ou leurs sels, selon la revendication 1, utilisés comme principes actifs pour des médicaments pour le traitement de l'hypertonie, de l'insuffisance cardiaque et/ou des troubles périphériques de la circulation.

## Revendications (Revendications pour l'(les) Etat(s) contractant(s) suivant(s): ES, GR)

1. Procédé de préparation de nouvelles pipérazinylalcoyl-3(2H)-pyridazinones de formule 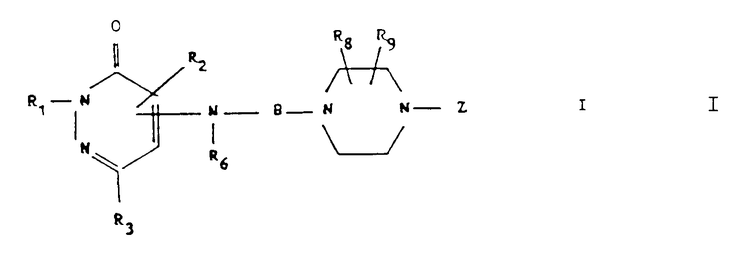 dans laquelle
R₁ représente l'atome d'hydrogène, un reste phényle, benzyle ou alcoyle en C₁-C₆ non substitué ou substitué une ou plusieurs fois par un groupe hydroxy, pipéridine, morpholine ou par un groupe NR₄R₅ dans lequel R₄ et R₅ peuvent être identiques ou différents et représentent l'atome d'hydrogène ou un groupe méthyle ou éthyle ;
R₂ et R₃ représentent l'atome d'hydrogène, un atome d'halogène, un groupe alcoxy en C₁-C₆ ou un groupe alcoyle en C₁-C₆, au moins un des restes R₂ ou R₃ signifiant l'atome d'hydrogène ;
R₆ représente l'atome d'hydrogène, un groupe alcoyle en C₁-C₄, phényle, benzyle ou phényléthyle ;
B représente un groupe alcoylène en C₁-C₇ non substitué ou substitue une ou plusieurs fois par un groupe hydroxy, alcoyle en C₁-C₄ ou par le groupe NR₄R₅, qui peut éventuellement être fermé en un composé alicyclique à 4 à 7 chaînons ;
R₈ et R₉, qui peuvent être identiques ou différents, représentent l'atome d'hydrogène ou un groupe alcoyle en C₁-C₆ ; et
Z représente un groupe phényle, naphtyle, pyridyle ou thiazolyle, non substitué ou substitué une ou plusieurs fois par un groupe alcoyle en C₁-C₆, alcoxy en C₁-C₆, benzyloxy, trifluorométhyle, halogéno, nitro, cycloalcoxy en C₃-C₇, alcoylthio en C₁-C₄, trifluorométhylthio ou par le groupe NR₄R₅,
et de leurs sels pharmaceutiquement utilisables, caractérisé en ce que :
a) on fait réagir un composé de formule 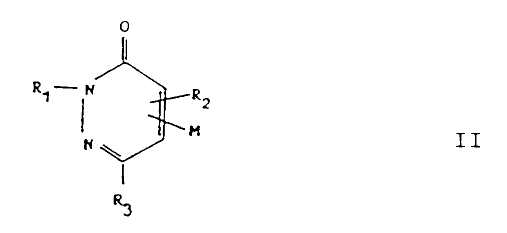 dans laquelle R₁, R₂ et R₃ sont définis comme indiqué ci-dessus et M signifie un groupe de départ, avec un composé de formule 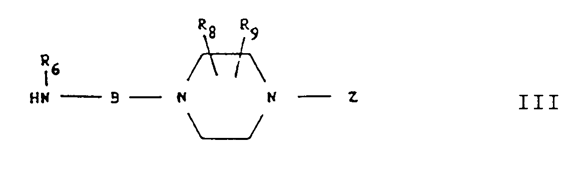 dans lequel R₆, B, R₈, R₉ et Z ont la signification indiquée ci-dessus, ou
b) on remplace, dans un composé de formule I, dans laquelle un des restes R₂ ou R₃ représente un atome d'halogène et les autres restes sont définis comme indiqué ci-dessus, l'atome d'halogène de R₂ ou R₃ par un atome d'hydrogène, par hydrodéshalogénation, ou
c) on fait réagir un composé de formule I, dans laquelle un des restes R₂ ou R₃ représente un atome d'halogène et les autres restes sont définis comme indiqué ci-dessus, avec un alcoolate de métal alcalin, l'atome d'halogène de R₂ ou R₃ étant transformé en un reste ayant la signification d'un groupe alcoxy en C₁-C₆, ou
d) on sépare R₁ par des acides dans un composé de formule I dans laquelle R₁ signifie un groupe i-propyle, sec.-butyle, t-butyle ou benzyle, et les autres restes sont définis comme indiqué ci-dessus, ou
e) on transforme une pyridazine de formule 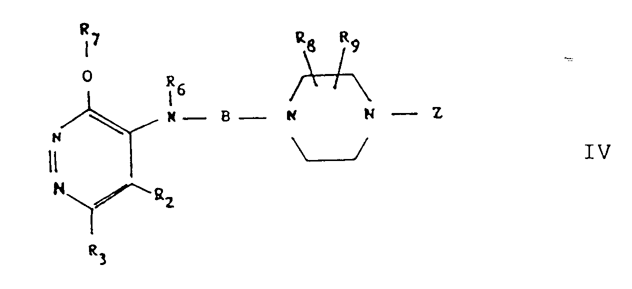 dans laquelle R₂, R₃, R₆, R₈, R₉ et Z sont définis comme indiqué ci-dessus et R₇ signifie un groupe alcoyle en C₁-C₆, avec séparation d'éther avec un acide, en la 3(2H)-pyridazinone correspondante, ou
f) on alcoyle un composé de formule I, dans laquelle R₁ représente l'atome d'hydrogène, un des restes R₂ ou R₃ représente un atome d'halogène et les autres restes sont définis comme indiqué ci-dessus, par réaction avec un réactif alcoylant en position 2 du cycle pyridazinique, et
g) si on le souhaite, on transforme un composé de formule I obtenu d'après a) à e) en ses sels pharmaceutiquement acceptables.

2. Procédé de fabrication de préparations pharmaceutiques, caractérisé en ce qu'on combine un composé de formule I ou un de ses sels, selon la revendication 1, avec des adjuvants et/ou des excipients ou des diluants galéniques usuels.

3. Utilisation des composés de formule I ou de leurs sels, selon la revendication 1, pour la préparation de médicaments pour le traitement de l'hypertonie, de l'insuffisance cardiaque et/ou des troubles périphériques de la circulation.
